# EUROPEAN PATENT SPECIFICATION

(11) **EP 2 707 483 B1**
(45) Date of publication and mention of the grant of the patent: **18.10.2017**
(21) Application number: 12721232.2
(22) Date of filing: 04.05.2012
(51) Int. Cl.: C12N 15/10

(54) **METHODS AND MATERIALS FOR NUCLEIC ACID MANIPULATION**
VERFAHREN UND MATERIALIEN ZUR MANIPULATION VON NUKLEINSÄUREN
PROCÉDÉS ET MATÉRIAUX POUR LA MANIPULATION DES ACIDES NUCLÉIQUES

(30) Priority: 09.05.2011 EP 11165367
(43) Date of publication of application: 19.03.2014
(73) Proprietor: RWTH Aachen University, 52062 Aachen (DE)
(72) Inventor: HÄFNER, Stefan, 67346 Speyer (DE); SCHWANEBERG, Ulrich, 52074 Aachen (DE); MARIENHAGEN, Jan, 52146 Würselen (DE); DENNIG, Alexander, 8010 Graz (AT); SHIVANGE, Amol, V., Maharashtra 413517 (IN)
(74) Representative: Eisenführ Speiser
(86) International application number: PCT/EP2012/058250
(87) International publication number: WO 2012/152691

(56) References cited:
- CN-B- 101 067 133
- BLANUSA MILAN ET AL: "Phosphorothioate-based ligase-independent gene cloning (PLICing): An enzyme-free and sequence-independent cloning method", ANALYTICAL BIOCHEMISTRY, vol. 406, no. 2, November 2010 (2010-11), pages 141-146, XP027259637, ISSN: 0003-2697
- DENNIG ALEXANDER ET AL: "OmniChange: The Sequence Independent Method for Simultaneous Site-Saturation of Five Codons", PLOS ONE, vol. 6, no. 10, October 2011 (2011-10), XP002681822, ISSN: 1932-6203
- ECKSTEIN F ET AL: "Phosphorothioates in molecular biology", TRENDS IN BIOCHEMICAL SCIENCES, ELSEVIER, HAYWARDS, GB, vol. 14, no. 3, 1 March 1989 (1989-03-01), pages 97-100, XP023669625, ISSN: 0968-0004, DOI: 10.1016/0968-0004(89)90130-8 [retrieved on 1989-03-01]
- ECKSTEIN F: "NUCLEOSIDE PHOSPHOROTHIOATES", ANNUAL REVIEW OF BIOCHEMISTRY, PALTO ALTO, CA, US, vol. 54, 1 January 1985 (1985-01-01), pages 367-402, XP000603155, ISSN: 0066-4154, DOI: 10.1146/ANNUREV.BI.54.070185.002055
- VROOM JONATHAN A ET AL: "Modular construction of plasmids through ligation-free assembly of vector components with oligonucleotide linkers", BIOTECHNIQUES, vol. 44, no. 7, June 2008 (2008-06), pages 924-926, XP002681823, ISSN: 0736-6205 cited in the application

## Description

### BACKGROUND

The present invention is concerned with the field of nucleic acid manipulation and particularly DNA manipulation, and uses thereof. Specifically, the invention pertains to methods involving the joining of nucleic acids and uses of such joined nucleic acids, for example for creating transformed microorganisms. Also, the invention pertains to materials useful in such methods.

There is a permanent need for nucleic acid manipulation methods. For example, analysis of the function of a nucleic acid or a protein encoded thereby, it is frequently helpful to create variants of said nucleic acid, particularly variants having a different base sequence. Likewise, for constructing efficient production strains or vectors it is frequently desired to combine several nucleic acid segments, for example to bring several genes or exons under the control of a desired regulatory element.

For practical reasons, it is frequently necessary not to work with a full length nucleic acid. Instead, smaller segments are obtained, modified, and later combined to obtain a modified full length nucleic acid. As a general rule, the number of segments increases with the number of modifications introduced in the nucleic acid. Also, it is common to manipulate a nucleic acid and insert it into a vector for cloning or expression purposes.

For such manipulation methods which involve the combination of two or more nucleic acids, the need to achieve a stable combination generally constitutes a problem. Stability of combination is particularly required for transformation of cells with the combined nucleic acid. During and after transformation, nucleic acids are subjected to stress, for example by the forced transfer into a cell, and by cellular enzymes designed to remove alien nucleic acids.

It has frequently been observed that combined nucleic acids which comprise nicks, i.e. a break in the phosphodiester backbone between adjacent nucleotides of one strand, are prone to disintegration. Also, nicked nucleic acids will achieve a low transformation efficiency. For example, Vroom and Wang (BioTechniques 2008, 924-926) describe a cloning experiment involving a vector and a number of inserts. The inserts and vector are joined by an overlap of 13-15 nucleotides, such that the vector and each insert will add nicks to the combined nucleic acid. The authors observe that the number of transformants decreases by an order of magnitude for each additional insert. For example, where only one insert was present 261 colonies are observed, where two inserts were simultaneously present only 16 colonies were observed, and for three inserts only 3 colonies were observed. Thus, in the construction of combined nucleic acids like plasmids the presence of nicks is to be avoided. And in particular for the construction of libraries of modified nucleic acids the presence of nicks has to be avoided, because a low transformation efficiency would impede or unacceptably encumber the generation of a library of transformants. Thus, it is tedious to construct a library of transformed cells harboring different nucleic acids, and for this reason it is presently difficult to analyze and compare the function of a large number of variants of a nucleic acid, or to combine several nucleic acid segments.

It is therefore desired to avoid the presence of nicks in combined nucleic acids, particularly where such nucleic acids are to be used for the generation of transformants. Thus, it is generally tried to ligate the phosphodiester backbone of combined nucleic acids, either by the action of a ligase or by synthesis of unnicked strands, e.g. by the action of a nucleic acid polymerase.

However, ligation is a notoriously cumbersome process and ligation efficiencies can vary for no obvious reasons. And amplification by nucleic acid polymerases also is considered an unwanted additional step and can lead to artifacts.

There is thus a need to provide a method for joining of multiple nucleic acids to remove or alleviate the above drawbacks of the prior art. The method should allow to combine at least three, preferably at least 4 and more preferably at least 5 nucleic acids. Also, the method should allow to achieve a high transformation efficiency. The method should allow to produce combined nucleic acids useful for the production of libraries of transformants. Also, the method should be reliable, fast and should not require expensive materials or devices.

### DETAILED DESCRIPTION OF THE INVENTION

According to the invention there is provided a nucleic acid joining method, comprising the steps of
a) providing a set of at least three nucleic acid fragments to be joined, wherein the nucleic acid fragments comprise pairwise compatible ends, and wherein at least two of the nucleic acid fragments are double stranded at least in the region of the respective compatible end, and wherein each counter-strand of the respective compatible end comprises nucleotides configured for non-enzymatic removal,
b) non-enzymatically removing of such nucleotides configured for non-enzymatic removal,
c) mixing of the nucleic acid fragments under hybridizing conditions,
   wherein the nucleotides configured for non-enzymatic removal comprise a phosphorothioate backbone, and wherein the compatible ends have a length of 5 to 25 nucleotides, and wherein the number of nucleotides configured for non-enzymatic removal for each compatible end region is 5 to 25, and wherein at least one fragment comprises a double-stranded section after performing of step b).

It has now surprisingly been found that by preparation of fragments as described above according to the invention, i.e. by non-enzymatically removing of nucleotides configured for non-enzymatic removal, joined nucleic acids can be obtained which can be transformed with high efficiency even though the respective joined nucleic acid comprises nicks. Specifically the method of the present invention abolishes the need for a ligation or polymerase reaction to achieve a combined nucleic acid transformable with high transformation efficiency. Thus the method of the present invention is advantageously suited to various applications requiring a step of transformation, as will be explained later.

Also, the present invention provides a surprisingly efficient way to join multiple nucleic acid fragments without having to use an enzyme to produce compatible ends and to join such compatible ends. The inventors have found that even the nucleic acids joined according to the present invention are further treated with a ligase, transformation efficiency does not significantly increase compared to the unligated joined nucleic acid. This was unexpected, as nicked nucleic acids have a much higher probability to disintegrate during or after transformation than non-nicked nucleic acids; it was thus expected that ligation is essential or at least helpful to achieve a high transformation efficiency. It is presently assumed by the inventors that the non-enzymatic removal of nucleotides provides an unexpectedly efficient process for generating single stranded compatible ends; this was particularly surprising as evolution should by now have developed nearly optimal enzymes for ligation and production of compatible ends. It was therefore surprising that transformation efficiencies according to the present invention could be the same or even much higher compared to processes involving enzymes in the creation and/or joining of nucleic acid fragments.

For the purposes of describing the present invention, the term "nucleic acid" denotes an ester of at least two nucleosides, wherein a nucleoside comprises a base preferably selected from purine and pyrimidine bases and more preferably selected from adenine, cytosine, guanine, thymine and uracil, and a pentose sugar moiety preferably selected from ribose and desoxyribose. Preferred nucleosides are thus adenosine, guanosine, cytidine and uridine for ribonucleosides, and deoxyadenosine, deoxycytidine, deoxyguanosine, thymidine and deoxyuridine for deoxyribonucleosides, which are particularly preferred nucleosides. Other nucleosides according to the invention are, for example, inosine, xanthosine, 7-methylguanosine, dihydrouridine and 5-methylcytidine.

Further preferred nucleotides are selected from the group consisting of 2'-Amino-2'-deoxyadenosine (2'-AdA), 3'-Amino-3'-deoxyadenosine (3'-AdA), 3'-Amino-3'-deoxyguanosine (3'-AdG), 5'-Amino-5'-deoxyadenosine (5'-NH₂-Ado), 5'-Amino-5'-deoxyguanosine (5'-NH_{Z}-Guo), 2-Amino-6-chloropurine riboside (2-NH₂-6-Cl-PuR), 5-Aminoimidazole-4-carboxamide-1-ß-D-ribofuranoside (AICAR / Acadesine / Z-riboside), 2',3'-O-*para*-Anisylideneguanosine (2',3'-O-pAnisyl-Guo), 2-Aza-1,N⁶-ethenoadenosine (2-Aza-ε-Ado), 8-Azidoadenosine (8-N₃-Ado), 8-Bromo-2',3',5'-triO-acetylguanosine (8-Br-2',3',5'-TAc-Guo), 8-Bromo-2'-deoxyadenosine (8-Br-dAdo ), 8-Bromo-2'-deoxyguanosine (8-Br-dGuo), 8-Bromo-2'-O-methylguanosine (8-Br-2'-O-Me-Guo), 8- Bromoadenosine, 8-Bromoguanosine, 6-Bromotubercidin / 8- Bromo-7-deazaadenosine (6-Br-Tu), 8-Bromoxanthosine (8-Br-Xao), N⁶-Carbamoylthreonyladenosine (t⁶-Ado), 8-Carboxyethylaminoadenosine (8-CEA-Ado), 2-Chloro-2'-deoxyadenosine (2-Cl-dAdo, Cladribine), 8-Chloro-2'-deoxyadenosine ( 8-Cl-d-Ado), 5-Chloro-2'-deoxycytidine (CldC), 8- Chloro-2'-deoxyguanosine (8-Cl-dGuo), 2-Chloroadenosine (2-Cl-Ado), 8-Chloroadenosine (8-Cl-Ado), 5-Chlorocytidine (5-Cl-C), 8-Chloroguanosine (8-Cl-Guo), 2-Chloroinosine (2-Cl-Ino), 8- Chloroinosine (8-Cl-Ino), 8-(4-Chlorophenylthio)-2'-O-methyladenosine (8-pCPT-2'-O-Me-Ado), 8-(4-Chlorophenylthio)-2'-O-methylguanosine (8-pCPT-2'-O-Me-Guo), 8-(4-Chlorophenylthio)adenosine (8-pCPT-Ado), 8- (4-Chlorophenylthio)guanosine (8-pCPT-Guo), 6-Chloropurine riboside, 5-Chlorouridine (5-Cl-U), 2'-Deoxy-1, N⁶-ethenoadenosine (ε-dAdo), 2'-Deoxy-2'-fluoroadenosine (2'-F-dAdo), 2'-Deoxy-8-hydroxyadenosine (8-OH-dAdo), 2'-Deoxy-8-hydroxyguanosine (8-OH-dGuo), 2'-Deoxyguanosine, 3'-Deoxyguanosine (3'-dG), 2'-Deoxyinosine, 3'-Deoxythymidine / 2', 3'-Dideoxythymidine (ddT), 3',5'-Diamino-3',5'-dideoxyadenosine (3',5'-DA-ddA), 5, 6-Dichloro-1-ß-D-ribofuranosyl benzimidazole (DRB), 2,6-Dichloro-2',3',5'-triacetylpurine riboside, 2,6-Dichloropurine riboside, 2',3'-Dideoxyadenosine (ddA), 2', 3'-Dideoxyguanosine (ddG), N²,N²-Dimethylguanosine (M²₂Guo), 1,N⁶-Ethenoadenosine (ε-Ado), 8- Hydroxyadenosine (8-OH-Ado), 8- Hydroxyguanosine (8-OH-Guo), 5-Iodotubercidin / 7- Deaza-7-iodoadenosine (5-I-Tu), 2',3'-Isopropylideneguanosine (2',3'-Ipr-Guo), 2', 3'-Isopropylideneinosine, 8-Mercaptoguanosine, 6- Mercaptopurine riboside, 8-(4-Methoxyphenylthio)adenosine (8-pMeOPT-Ado), 2'-O-Methyladenosine (2'-O-Me-Ado), 2'-O-Methylguanosine, 6-Methylmercaptopurine riboside, 2- Methylthioadenosine (2-MeS-Ado), 8- Nitroguanosine (8-NO₂-Guo), 8-Phenylthioadenosine (8-PT-Ado), Purine riboside (PuR) / Nebularine, 5'-[(2,2,5,5-Tetramethyl-3-pyrrolin-1-oxyl-3-carboxylamino)adenosine (5'-TMPO-Ado), 2',3',5'-Tri-O-benzoylguanosine (2',3',5'-TBnz-Guo), 2',3',5'-Triacetylinosine, N²,N²,7-Trimethylguanosine (^{m2,2,7}G / TMG), and, for fluorescent nucleotides, 2-Aminopurine riboside- 3',5'-monophosphate (2-NH₂-cPuMP), 2- Aminopurine riboside-5'-O-triphosphate (2-NH₂-PuTP), 2-Aza-1,N⁶-ethenoadenosine (2-Aza-ε-Ado), 2-Aza-1,N⁶-ethenoadenosine-3',5'-cyclicmonophosphate (2-Aza-ε-cAMP), 8-Azido-1, N⁶-ethenoadenosine-3',5'-cyclic monophosphate (8-N₃-ε-cAMP), 8- Azido-N⁶-(2-[DY-547]- aminoethyl)adenosine-3',5'-cyclic monophosphate (8-N₃-6-[DY-547]-AE-cAMP), 6,7-Bis(carboxymethoxy)coumarin-4-yl)methyl-adenosine-3',5'-cyclic monophosphate (BCMCM-caged cAMP), [6,7-Bis(carboxymethoxy)coumarin- 4-yl]methyl- 8- bromoadenosine- 3', 5'- cyclic monophosphate (BCMCM-caged 8-Br-cAMP ), [6, 7- Bis(carboxymethoxy)coumarin- 4- yl]methyl- 8- bromoguanosine- 3', 5'- cyclic monophosphate (BCMCM-caged 8-Br-cGMP), [6, 7- Bis(carboxymethoxy)coumarin- 4-yl]methyl- guanosine- 3', 5'- cyclic monophosphate (BCMCM-caged cGMP), 2'- Deoxy-1, N⁶- ethenoadenosine (ε-dAdo), 2'- Deoxy- 1, N⁶- ethenoadenosine- 5'- O-monophosphate (ε-5'-dAMP), 2'-Deoxy-1,N⁶-ethenoadenosine-5'-O-triphosphate (ε-dATP), 2'-Deoxy-3'-O-(N'-methylanthraniloyl)adenosine-5'-O-diphosphate (MANT-dADP), 2'-Deoxy-3'-O-(N'-methylanthraniloyl)adenosine-5'-O-monophosphate ( MANT-5'-dAMP), 2'-Deoxy-3'-O-(N'-methylanthraniloyl)adenosine-5'-O-triphosphate (MANT-dATP), 2'-Deoxy-3'-O-(N'-methylanthraniloyl)guanosine-5'-O-diphosphate ( MANT-dGDP), 2'-Deoxy-3'-O-(N'-methylanthraniloyl)guanosine-5'-O-monophosphate (MANT-5'-dGMP), 2'-Deoxy-3'-O-(N'-methylanthraniloyl)guanosine-5'-O-triphosphate (MANT-dGTP), 2'-, 2"-O-(Di-N'-methylanthraniloyl)-cyclic diguanosine monophosphate (Di-MANT-c-diGMP), 8-(2-[DY-547]-aminoethylthio)-2'-O-methyladenosine-3',5'-cyclicmonophosphate (8-[DY-547]-AET-2'-O-Me-cAMP), 8-(2-[DY-547]-aminoethylthio)guanosine-3',5'-cyclicmonophosphate (8-[DY-547]-AET-cGMP), 8-(2-[DY-647]-aminoethylthio)guanosine-3',5'-cyclic monophosphate (8-[DY-647]-AET-cGMP), 1,N⁶- Ethenoadenosine-3',5'-cyclic monophosphate (ε-cAMP), 1, N⁶-Ethenoadenosine-5'-O-diphosphoribose (ε-ADPR / ε-ADP-ribose), 1,N⁶-Ethenoadenosine-5'-O-diphosphoribose phosphate (ε-ADPRP), 1,N⁶-Ethenoadenosine-5'-O-monophosphate (ε-5'-AMP), 1, N⁶- Ethenoadenosine- 5'- O-triphosphate (ε-ATP), 8-(2-[Fluoresceinyl]aminoethylthio)adenosine-3',5'-cyclic monophosphate (8-[Fluo]-cAMP), 8-(2-[Fluoresceinyl]aminoethylthio)guanosine-3',5'-cyclic monophosphate (8-[Fluo]-cGMP), 2'-O-(6-[ Fluoresceinyl]aminohexylcarbamoyl)- cyclic diguanosine monophosphate (2'-Fluo-AHC-c-diGMP), 2'-(6-[Fluoresceinyl]aminohexylcarbamoyl)adenosine-3',5'-cyclic monophosphate (2'-Fluo-AHC-cAMP / FAM-cAMP), 2'- / 3'-O-(6-[ Fluoresceinyl]aminohexylcarbamoyl)adenosine-5'-O-monophosphate (2'-/3'-Fluo-AHC-5'-AMP / FAM-AMP), 2'-(6-[Fluoresceinyl]aminohexylcarbamoyl)guanosine-3',5'-cyclic monophosphate (2'-Fluo-AHC-cGMP / FAM-cGMP), 2'-O-(N-Methylanthraniloyl)adenosine- 3', 5'- cyclic monophosphate (MANT-cAMP), 2'-/3'-O-(N'-Methylanthraniloyl)adenosine-5'-O-diphosphate (MANT-ADP), 2'-/3'- O-(N'-Methylanthraniloyl)adenosine-5'-O-monophosphate (MANT-5'-AMP), 2'-/3'-O-(N-Methylanthraniloyl)adenosine-5'-O-triphosphate (MANT-ATP), 2'-O-(N'-Methylanthraniloyl)cytidine- 3',5'-cyclic monophosphate (MANT-cCMP), 2'-O-(N-Methylanthraniloyl)guanosine-3',5'-cyclic monophosphate (MANT-cGMP), 2'-/3'-O-(N'-Methylanthraniloyl)guanosine-5'-O-diphosphate (MANT-GDP), 2'-,3'-O-(N'-Methylanthraniloyl)guanosine-5'-O-monophosphate (MANT-5'-GMP), 2'-/3'-O-(N'-Methylanthraniloyl)guanosine-5'-O-triphosphate (MANT-GTP), 2'- / 3'-O-(N'-Methylanthraniloyl)guanosine-5'-O-[(β,γ)-imido]triphosphate (MANT-GppNHp), 8- (4-[N-Methylanthraniloyl]aminobutylamino)adenosine-3',5'-cyclic monophosphate (8-MABA-cAMP), N⁶-(6-[N-Methylanthraniloyl]hexylamino)adenosine-3',5'-cyclic monophosphate (6-MAH-cAMP), β-Nicotinamide-1,N⁶-ethenoadenine dinucleotide (ε-NAD+), β-Nicotinamide-1,N⁶-ethenoadenine dinucleotide phosphate (ε-NADP+), β-Nicotinamide- N⁶-(2-(6-[fluoresceinyl]aminohexanoyl)aminoethyl)adenine dinucleotide ( 6-Fluo-10-NAD+), Nicotinic acid-1,N⁶-ethenoadenine dinucleotide phosphate (ε-NAADP), 8-(2-[7-Nitro-4-benzofurazanyl]aminoethylthio)adenosine- 3',5'-cyclic monophosphate (8-NBD-cAMP), 8-(2-[7-Nitro-4-benzofurazanyl]aminoethylthio)guanosine-3',5'-cyclic monophosphate (8-NBD-cGMP), 8-[Pharos-450]-adenosine-3',5'-cyclic monophosphate (8-[cp-450]-cAMP), 8-[ Pharos-450]-guanosine-3',5'-cyclicmonophosphate (8-[cp-450]-cGMP), 8-[Pharos-575]-2'-O-methyladenosine-3',5'-cyclic monophosphate (8-[(ϕ-575]-2'-O-Me-cAMP), 8-[Pharos-575]-adenosine-3',5'-cyclic monophosphate (8-[ϕ-575]-cAMP), 8-[ Pharos-575]-guanosine-3',5'-cyclic monophosphate (8-[ϕ-575]-cGMP), 2'-O-(2-[ Tetramethylrhodaminyl]aminoethylcarbamoyl)guanosine-3',5'-cyclic monophosphate ( 2'-TAMRA-AEC-cGMP), 2'-O-(6-[ Tetramethylrhodaminyl]aminohexylcarbamoyl)guanosine-3',5'-cyclic monophosphate ( 2'-TAMRA-AHC-cGMP).

According to the present disclosure the ester bond of the nucleosides preferably is a phosphodiester bond, or is a modified phosphodiester bond as described below, wherein phosphorothioates are most preferred. A nucleoside with such covalently attached phosphate or modified phosphate group is termed nucleotide for the purposes of describing the present invention. The phosphate or modified phosphate group is preferably covalently attached to the 3' carbon atom of the pentose sugar of the nucleotide.

The term "nucleic acid fragment" (or simply "fragment") in view of the present invention denotes a nucleic acid intended for combination with another nucleic acid, which is likewise according to the present invention termed a fragment. It is possible but not required that a nucleic acid fragment results from the digestion of a prior larger nucleic acid; instead, a nucleic acid fragment can also be the direct result of a de novo synthesis, for example via a polymerase chain reaction or other mechanism.

Preferably the nucleic acid or nucleic acid fragment is a RNA or a DNA, additionally comprising nucleotides configured for non-enzymatic removal, which are nucleotides comprising a phosphorothioate group. The nucleic acid used in the present invention in a joining method of the present invention can in particular be a double stranded RNA or DNA, and the joining method of the present invention allows to join an RNA fragment to a DNA fragment to obtain a mixed nucleic acid.

Two nucleic acids and nucleic acid fragments can hybridize via hydrogen bonds of their compatible bases. The hybridized nucleic acids then form the two strands of a double-stranded nucleic acid. For the purposes of describing the present invention, there is generally made no explicit distinction between single- and double-stranded nucleic acids. Also, nucleic acids according to the present invention can be single stranded over their whole length, double-stranded over their whole length, or contain single- and double-stranded sections. Particularly, a nucleic acid fragment according to the present invention can be fully single stranded, or can contain one or more double-stranded section. Preferably, the nucleic acid fragments according to the present invention comprise a single stranded section at both of their ends after step b); more preferably they are completely single stranded, have a single-stranded end section extending to their 5' end and a single-stranded section extending to their 3' end, or are double-stranded with a single-stranded end section extending to the 5' end of each strand.

A single-stranded end section of a first nucleic acid is termed a compatible end if, under hybridizing conditions, the end hybridizes with an end section of a second nucleic acid used in a hybridizing step of a method according to the present invention, wherein the first and second nucleic acid do not consist of exactly matching counter-strands of each other. Preferably, pairwise compatible ends (also called cohesive or sticky ends) have a nucleotide sequence exactly matching each other according to standard base pairing rules, such that adenine corresponds to thymine or uracil, and vice versa, and guanine corresponds to cytosine, and vice versa. However, the invention is not limited in this respect; nucleic acid strand sections are also considered compatible with each other if they mismatch on at most 3 positions provided that at least 5 exactly matching positions extend consecutively starting from the mismatched position most distant from the respective end.

Hybridizing conditions according to the present invention are conditions which allow formation of double-stranded nucleic acids via hydrogen bonds of corresponding base pairs. Hybridizing conditions will thus vary according to the base sequence of the nucleic acids. Hybridization according to the invention is performed in water, preferably in a buffered medium. Most important parameter of hybridizing conditions is the water temperature, also termed hybridization temperature. The following hybridization temperatures are preferred hybridization conditions:

| Length of single-stranded section to be hybridized [nt] | Temperature [°C] |
|---|---|
| 5 to 10 | 10 to 50 |
| 11 to 15 | 11,3 to 69,3 |
| 16 to 20 | 28,4 to 78,7 |
| 21 to 25 | 37,3 to 84 |

The skilled person can thus choose hybridizing conditions according to the specific needs of the nucleic acid fragments to be joined. As a general rule, a higher temperature will result in more stringent hybridization, such that base mismatches will be reduced. Also, higher temperatures generally facilitate breaking of hydrogen bonds between adenine and thymine or uracil, such that nucleic acid double strands having a high content of these bases will not form readily or will be reduced. The skilled person can thus adjust temperature according to the required specificity of base pairing in view of the content of adenine, thymine and uracil of the respective nucleic acids. Preferably, the hybridizing conditions are chosen such that compatible ends of nucleic acids hybridize without mismatches.

In view of the advantages of the present invention, it is highly preferred that the nucleic acid joining method is not followed by a step of ligation or amplification of the joined nucleic acid. Particularly, the present invention allows to use the nucleic acid joined according to the present invention directly for transformation of cells, as will be described below in more detail. Even though it is possible to ligate or amplify the joined nucleic acid, best use of the invention is made without such ligation or amplification step, because the possibility to avoid of the additional step without compromising transformation efficiency is considered an important advantage of the present invention as described above.

According to the invention, in step a) a set of at least three nucleic acid fragments to be joined are provided. The fragments are structurally related to each other by comprising compatible ends which allow hybridization of the respective compatible ends via hydrogen bonds as described above. It is not necessary and frequently not even desired that each nucleic acid fragment comprises ends compatible with every other nucleic acid fragment. Instead, it is preferred that the compatible ends are chosen such that each end of each nucleic acid fragment is compatible only with a compatible end of one other nucleic acid fragment. This way, an unambiguous chain relationship between the nucleic acid fragments can be maintained, such that for example in a 5'-3' direction a fragment A having a first nucleobase sequence is compatible with a fragment B having a second nucleobase sequence, which in turn is compatible via its other end section with a fragment C having a third nucleobase sequence, etc. If an end of a nucleic acid fragment is compatible to an end of more than one other nucleic acid fragments, the fragments can be joined arbitrarily such that there is a high probability that not all joined nucleic acids obtained in step c) will comprise all nucleic acid fragments provided in step a). This is preferred only for such applications where the exact composition of the joined nucleic acids obtained in step c) is not decisive, i.e. where it is not required that every nucleic acid fragment is present in every joined nucleic acid, or where the number of occurrences of nucleic acid fragments in each joined nucleic acid can be greater than 1.

Preferably, in step a) a set of 3 to 8 nucleic acid fragments to be joined are provided, wherein, as indicated above, preferably an unambiguous chain relationship is maintained via the compatible ends. Even though the maximum number of fragments is not limited, so far best results have been obtained by methods joining not more than 8 fragments by a method according to the present invention. More preferably, the number of nucleic acid fragments provided in step a) is 4 to 16, more preferably 4 to 10, and even more preferably 5 to 8. In view of the above described publication by Vroom and Wang, it was particularly surprising that such high number of fragments can be joined and used for transformation with high transformation efficiency. Due to the very low number of transformants obtainable by the method of Vroom and Wang, such method of the prior art is not industrially applicable or would impose an undue burden on the skilled person when trying to obtain joined nucleic acids. Also, in view of a publication by Pachuk et al. (Chain reaction cloning: a one step method for directional ligation of multiple DNA fragments, Gene 243 (2000), 19-25), the skilled person had to expect that only a very complicated protocol would be sufficient to provide any ligation of 6 fragments, whereas according to the present invention a very simple protocol is sufficient. Also, Pachuk et al. did not publish the transformation efficiency, and they observed that only 50% of all clones comprised the desired construct. Thus, the skilled person had to expect that such method would not be useful to obtain a library of different nucleic acids, as would be required for any sort of nucleic acid shuffling like gene shuffling, exon shuffling or domain shuffling. Even more preferably, the number of fragments provided in step a) is at least 5. According to the method disclosed by Vroom and Wang, the skilled person could not have a reasonable expectation of succeeding with a joining method involving at least 5 fragments without undue burden, because the authors describe that even by joining four fragments no practically number of transformants could be obtained. For the same reason, a method according to the invention is preferred wherein the number of nucleic acid fragments provided in step a) is at least 6, and even more preferably is at least 7.

The composition of the nucleic acid fragments in view of their base sequence is not restricted other than by the requirement that they comprise compatible ends. The fragments may comprise one or more elements selected from hairpin loops, restriction enzyme binding sites, transcription factor binding sites, recombination sites, origins of replication, scaffold attachment sites, promoters, transcription terminators, other noncoding nucleic acid sequences, metagenomic nucleic acid sections (cf. for example Handelsman J. et al., Chemistry & Biology 1998, 5:R245-249), exons, introns, genes and operons. According to the invention, a fragment may for example be of such composition that it is useful (after joining) as a vector for transforming a target cell. A vector generally comprises at least one origin of replication for allowing replication of the vector and any nucleic acid segment inserted therein by a selected target cell. Preferably, a vector also comprises a means for detecting its presence in the target cell, preferably a reporter gene and most preferably a gene for producing a dye, light and/or resistance against a selected stress factor, preferably antibiotics resistance. Preferably, in step a) at least one of the fragments is the fragment of a vector, such that in step c), a nucleic acid is obtained which can be replicated as described above. Also preferably, in step a) at least two fragments are provided which, after joining, allow the thus obtained nucleic acid to be replicated. In such case, the vector is essentially made up by the two or more fragments. Vectors according to the present invention preferably are plasmids, bacterial artificial chromosomes, yeast artificial chromosomes or viral vectors. As indicated above, however, the joined nucleic acid does not have to be or comprise a vector at all.

Also, the length of the fragments is not restricted other than by the requirement that the fragments comprise compatible ends. Preferably, the fragments have a length of 30 to 1,150,000 nucleotides, more preferably 30 to 50,000 nucleotides. In particular, the present invention advantageously allows to join even very large fragments for example as described by Gibson et al. (Creation of a Bacterial Cell Controlled by a Chemically Synthesized Genome; Science 329 (2010), 52-56) at a practically useful transformation efficiency, particularly to create a library of different transformants. Such joining of large fragments previously was very tedious and could only be achieved fragment by fragment, as described in the cited prior art.

According to the invention the fragments comprise mutually compatible ends. As indicated above, the base sequence of the compatible ends are chosen in view of the type of joined nucleic acid to be obtained in step c). Thus, the compatible ends may or may not be chosen to maintain an unambiguous chain relationship. Also, it is not required that each end of each fragment is compatible with at least one end of another fragment. Of course, only such fragments are joined in a method according to the present invention which comprise at least one end compatible to at least one end of another nucleic acid fragment. Thus, if for all fragments all ends are compatible with at least one end of another fragment, a circular nicked nucleic acid can be obtained in step c). For the purposes of providing nucleic acids ready for transformation and cloning, this is preferred. If at least one fragment comprises only one end compatible with another fragment, a linear nucleic acid can be obtained in step c). Such linear nucleic acids can, for example, be used to construct nucleic acid chips, which comprise an array of nucleic acids bound to the surface of a carrier chip. Also, they could be used as a DNA probe in blotting or as antigens. And also, linear nucleic acids could be used as templates for transcription and in vitro protein expression. Preferably, linear nucleic acids obtained in step c) comprise a 5'- and/or 3'-end modification, preferably biotin or a fluorescence label.

According to the invention at least two of the nucleic acid fragments are double stranded at least in the region of the respective compatible end, and each counter-strand of the respective compatible end comprises nucleotides configured for non-enzymatic removal. Also, these fragments are preferably double stranded such that after joining of the respective compatible ends no gaps are left in the joined nucleic acid other than nicks. Preferably, however, all fragments provided in step a) are double stranded at least in the region of the respective compatible end, and each counter-strand of the respective compatible end comprises nucleotides configured for non-enzymatic removal. This way it is particularly easy to prepare all nucleic acid fragments to be used according to the present invention, because they may all be prepared by the same way, for example by amplification with modified primers as described below. However, some fragments like vectors can also be obtained easily in a linearized form from commercial sources. Thus, a method according to the present invention is also preferred wherein all but one nucleic acid fragments are double stranded at least in all regions of their respective compatible ends, and each counter-strand of the respective compatible end comprises nucleotides configured for non-enzymatic removal.

Preferably, the nucleotides configured for non-enzymatic removal are located in the 5' end region of a nucleic acid strand (hereinafter: marked strand). This way, the marked strand can be easily synthesized, for example by using a primer comprising the nucleotides configured for non-enzymatic removal and extending the primer by a template driven amplification reaction, e.g. by PCR. The respective nucleic acid fragment will then, after removal of the nucleotides configured for non-enzymatic removal as described below, have a region which is single-stranded at the respective 3' end of the counter-strand.

The nucleotides configured for non-enzymatic removal can also be located in the 3' end region of the marked strand. In such case, the nucleotides configured for non-enzymatic removal preferably comprise a phosphothiolisation. For example, a first fragment could comprise only nucleotides configured for non-enzymatic removal located in the 3' end region, and a second and third fragment could comprise nucleotides configured for non-enzymatic removal located in the respective 3' and 5' end region. This way, there is only one way to join the three fragments, thereby achieving a great control over the joining process.

According to the invention, nucleotides configured for non-enzymatic removal are non-enzymatically removed in step b). It is understood that nucleotides not configured for non-enzymatic removal should not be removed in step b), otherwise no nucleic acid would remain for joining in step c). Particularly preferred methods and reagents for non-enzymatic nucleotide removal are described hereinafter. It is preferred to treat all fragments in a single reaction mixture in step b), because such method is particularly easy and fast to perform. However, alternatively some or all fragments can be treated in separate reaction mixtures. Such individual treatment allows to maintain a small reaction mixture volume, thereby maintaining a high heat exchange efficiency. Also, the individual fragments can be measured individually in the mixing step c) of the joining method of the present invention, thereby avoiding any preference or bias in view of one or more fragment(s).

During or after step b), the nucleic acid fragments are mixed under hybridizing conditions. If in step b) not all fragments are kept in a single reaction or storage mixture, then they are now combined. In such cases, the fragments can be added sequentially to a hybridization mixture such that the number of fragments increases one by one. If the length of the fragments differs by more than 20 % relative to the longest nucleic acid strand to be joined in step c), then sequential addition of the smaller fragments to the larger fragments provides a higher yield of correctly joined nucleic acids. This sequential addition of smaller fragments to larger ones is particularly useful to ensure that a desired sequence of fragments is achieved, for example by saturating the compatible ends of the larger fragment with the desired subsequent smaller fragment, and only then adding the next fragment to again saturate the corresponding compatible end of the joined nucleic acid with the desired next fragment. When a vector fragment is to be joined to a chain of smaller inserts, it is thus preferred to add the first fragment to be joined via hybridization with the vector fragment, and then to add the consecutively next fragment until all fragments are present in the hybridization mixture. For fragment shuffling and recombination, however, this may not be required, as in such cases a high level of randomness is generally desired.

However, it is also possible first to mix all fragments in a single mixture, then to break up any potentially formed hybridization, and finally to apply hybridizing conditions. For example, the fragments can be mixed in a single mixture kept at 4 °C to ensure that no significant spontaneous separation of hybridized fragments will occur. After mixing of all fragments, the mixture is heated to break up hybridizations of the fragments, preferably by raising the temperature of the mixture to 70-80 °C for 3 minutes. If lower temperatures are employed, more time is required for breaking up the hybridization of fragments, if higher temperatures are employed, spontaneous degradation of fragments can occur. After heating, the mixture temperature is lowered to the hybridization temperature as indicated above, and the mixture is maintained at that temperature for a preselected time, preferably for 5 min at 20 °C. Surprisingly, with such mixing, heating and hybridizing protocol no preferential hybridization of fragments occurs. This is very advantageous, as the method of the present invention thus allows preparing a fair mixture of joined fragments.

After step c), joined nucleic acid products are obtained which can, for example, be used for transformation of a target cell to construct a library of transformants without the need to remove nicks in the products by ligation or nucleic acid amplification.

The compatible ends according to the invention have a length of 5 to 25 nucleotides, more preferably 7 to 25 nucleotides, even more preferably 10 to 18 nucleotides and most preferably 11 to 14 nucleotides. For compatible ends having a length of at least 7 nucleotides, hybridization at temperatures of 20-25 °C is highly selective such that an unambiguous chain of fragments can be defined by the compatible ends of the nucleic acid fragments. Thus, hybridization in step c) can be performed at standard laboratory room temperatures and does not require additional devices for heating or cooling the mixture of hybridizing fragments.

According to the invention the number of nucleotides configured for non-enzymatic removal for each compatible end region is 5 to 25, more preferably 7 to 25, even more preferably 10 to 18 and most preferably 11 to 14. It is thus not required that every nucleotide of the counter-strand of the compatible end is configured for non-enzymatic removal. For example, if the counter-strand comprises in the compatible end region a number of individual standard nucleotides dispersed between nucleotides configured for non-enzymatic removal, then such standard nucleotides not configured for non-enzymatic removal will not bind so tightly to the compatible end that they would impede correct hybridization in step c). It is thus preferred that at most 6, more preferably at most 4, even more preferably at most 2 nucleotides are comprised in the section forming the counter-strand section or region of the compatible end. Even more preferably, all nucleotides of a fragment forming the counter-strand of a compatible end, i.e. the counter-strand region of the compatible end, are configured for non-enzymatic removal.

As described above, preferably at least one fragment comprises a double-stranded section after performing of step b), and even more preferably all fragments comprise a double-stranded section after performing of step b). This way the joined nucleic acid does not comprise gaps other than nicks in the phosphodiester backbone. The absence of gaps other than nicks advantageously increases the transformation efficiency of such joined nucleic acids.

According to the invention, it is preferred that in step c) at least two nucleic acid fragments are mixed in a molar ratio of 20:1 to 1:20, even more preferably 1:1 to 1:10 and even more preferably 1:1 to 1:8. Preferably, the smaller fragment is added in surplus.

Preferably, all nucleotides configured for non-enzymatic removal are configured such that only one mechanism of removal of all such nucleotides needs to be employed. This constraint adds to the ease and high speed of the joining method of the present invention.

According to the present invention, nucleotides configured for non-enzymatic removal comprise a phosphorothioate group. This group is covalently attached to the sugar moiety of adjacent nucleotides such that the phosphate group of the nucleic acid backbone is replaced by a phosphorothioate group at the respective nucleotide. Synthesis and alkylation of phosphorothioate nucleotides and breakage of intermediates is described in publications by Eckstein et al., for example in Accounts of Chemical Research 1979, 204-210, Ann. Rev. Biochem. 1985 (54), 367-402 and Science 1988 (240), 1520-1522. Such nucleotides are not significantly more expensive than standard nucleotides, they can be used in polymerization reactions, they allow to form stable double-stranded nucleic acids and yet allow to be easily removed by hydrolysis during step b) of the joining method of the present invention.

According to the present invention, nucleotides comprising a phosphorothioate group are preferably removed non-enzymatically by exposure to elementary iodine and a molar surplus of an alcohol selected from ethanol (most preferred), isopropanol, and derivatives thereof comprising electron drawing moieties at the C1 or C2 carbon atom, for example perfluoroethanol, perchloroethanol, perbromoethanol, 2-fluoroethanol, 2-chloroethanol, 2-bromoethanol, perfluoroisoproyl alcohol, perchloroisopropyl alcohol, perbromoisopropyl alcohol, 2-fluoroisopropyl alcohol, 2-chloroisopropyl alcohol, and 2-bromoisopropyl alcohol. Preferably the nucleotides are exposed to a mixture comprising elementary iodine and ethanol, wherein ethanol is present in a molar surplus. Preferably, the mixture comprises elementary iodine and ethanol in a molar ratio of 1 (iodine): 80 (ethanol) to 1 (iodine) : 500 (ethanol), more preferably 1:120 to 1:240, and most preferably 1:160 to 1:180. At such ratios, hydrolysis of the phosphorothioate nucleotides occurs while hydrolysis of the ordinary phosphate backbone is substantially avoided. Also, the phosphorothioate groups are exposed to iodine and ethanol preferably at a pH of 8-9.5, even more preferably of 8.8 to 9.1 and at a temperature of 65-75 °C, preferably of 70 °C. It has now been found that under such conditions, presence of standard nucleotides as described above does not impede the removal of nucleotides from compatible end regions. This is another significant advantage of the present invention, because it allows to disperse a limited number of standard nucleotides as explained above in the compatible end regions.

Blanusa et al, Analytical Biochemistry 2010, 141-146 describe a phosphorothioate-based ligase independent gene cloning method. The method relies on the non-enzymatic removal of phosphorothioate modified nucleotides to generate compatible sticky ends of a vector and an insert. The authors describe that the method discussed therein is applicable for directed protein evolution and development of high-throughput screening systems. However, the document does not disclose or suggest that three or more fragments could be joined and transformed with high efficiency to create a cloned library of different nucleic acids. Also, in view of the above mentioned publication of Vroom and Wang, the skilled person could not reasonably expect that unligated nucleic acids comprising more than 4 nicks would be stable enough under standard laboratory conditions for handling, e.g. storage for transformation at some later time.

The nucleic acid joining method of the present invention is particularly useful for altering a given nucleic acid, for example to study the function of said nucleic acid by studying the function of variants of said nucleic acid. According to the invention there is thus also provided a nucleic acid alteration method, comprising the steps of
a) providing a template nucleic acid, and providing primers for amplification of a template section of the template nucleic acid, wherein one or all primers have nucleotides configured for non-enzymatic removal,
b) amplification of the template section of the template nucleic acid to obtain a nucleic acid fragment,
c) repeating steps a) and b) to obtain at least two nucleic acid fragments,
d) performing a nucleic acid joining method according to any of the previous claims on the nucleic acid fragments of step c), and, where only two nucleic acid fragments are obtained in step c), introducing at least one further nucleic acid fragment,
wherein the nucleotides configured for non-enzymatic removal comprise a phosphorothioate backbone, and wherein the compatible ends have a length of 5 to 25 nucleotides, and wherein the number of nucleotides configured for non-enzymatic removal for each compatible end region is 5 to 25, and wherein at least one fragment comprises a double-stranded section after performing of step b).

As indicated above, the joining method of the present invention is particularly useful for producing a large number of variants of one or more given nucleic acids, or for combining or rearranging nucleic acid segments. Preferred uses of the joining method, and also of the additional steps of the alteration method of the present invention, will be described later in this description and examples.

The primers comprising nucleotides configured for non-enzymatic removal provided in step a) correspond to the compatible end counterstrands of the joining method of the present invention. Preferably, the primers do not comprise any base pair mismatches to the respective template nucleic acid. However, the primers may also consist of a 5' end section comprising the nucleotides for non-enzymatic removal (preferably phosphorothioate nucleotides as described above) and an adjacent 3' end section free of nucleotides for non-enzymatic removal. In such case, the 5' end section corresponds to the counterstrand of a compatible end according to step a) of the joining method of the present invention. This 5' end section may have but does not have to have any significant homology to the template nucleic acid; instead, the 3' end section of the primer is chosen to hybridize with the template nucleic acid for amplification in above step b) to obtain a nucleic acid fragment. Preferably, this 3' end section has a length of at least 15 nucleotides, even more preferably 18-35 nucleotides, most preferably at least 20 nucleotides.

The joining and alteration method of the present invention allows to create a new nucleic acid with fragments related to one or more template nucleic acids. It is generally possible to use identical primers to obtain identical nucleic acid fragments in above step c) of the alteration method. Preferably, however, the primers are chosen to produce different nucleic acid fragments in above step c). This is preferably achieved by (a) using primers hybridizing with different positions of a template nucleic acid, or (b) using primers hybridizing with identical positions of a template nucleic acid, wherein the nucleobase sequence of the primers differ from one another, or (c) using different template nucleic acids, or (d) using different template nucleic acids and primers hybridizing with the respective template nucleic acid, wherein such hybridization contains one or more base mismatches.

An advantage of variant (a) is that the number of nucleic acids to use in the method of the present invention is comparatively low. For example, the primers may be chosen to copy a regulatory element and a gene sequence from a single template nucleic acid, where the regulatory element does not exert an influence on the respective gene in the template nucleic acid. The method of the present invention then allows to join a vector fragment, the fragment comprising the regulatory element and the fragment comprising the gene to bring the gene under the control of the regulatory element in the joined nucleic acid. Thus, the present invention allows to rearrange sections of a given template nucleic acid; preferred purposes of such rearrangements are described later.

To further increase the variability, primers according to variant (b) for a first and a second nucleic acid fragment hybridizing to the same template strand and position may have different nucleobase sequences. Thus, it is possible to create copies of a nucleic acid template section differing from said template section in a number of positions. This is useful to create libraries for example of point mutations. Of course, it is possible to combine variants (a) and (b) by using primers for first and second nucleic acid fragments which hybridize with different sections the template, and wherein this hybridization comprises one or more base mismatches.

According to variant (c), different template nucleic acids are used to prepare a first and second (or further) nucleic acid fragment. Depending on the templates, the nucleobase sequence of the primers can be identical, for example where such primers bind to identical or highly similar sequences like conserved sequences. In this case, the fragments may still have differing nucleobase sequences due to differences in the template sequence between the primer hybridization sections. Also according to variant (c), the primer sequences for a first and second fragment can be different from one another such as to allow them to hybridize without mismatches to template sections having low sequence homology. The use of different templates allows for example to combine heterologous nucleic acid sections. Also, it is possible to exchange for example a section of a nucleic acid sequence by creating a fragment to copy either side of the section, and to provide a third fragment which is to function as the replacement of the section in question. When the fragments are joined according to the invention, the resulting nucleic acid product will comprise the third fragment instead of the section to be replaced.

It is according to variant (d) also possible to use primers for a first and second fragment having different sequences, for example to let these primers hybridize to template sections having a very low homology or to introduce additional differences like point mutations as described above.

One use of the joining method of the present invention thus allows to create a joined nucleic acid product differing from a template by at least one point mutation. The point mutation(s) can be located in the section corresponding to a primer in step a) of the alteration method according to the present invention. This location of point mutations is preferred, because of the ease of synthesis of correspondingly mutated primers. Also, the joined nucleic acid product may comprise more than one point mutation relative to a template, such that one or more fragments contribute one or more point mutations to the final joined nucleic acid product. This is particularly preferred to create libraries of variants of a template, wherein more than one position of the template is mutated relative to the template. For example, the joined nucleic acid product can be in the form of a mixture of product species which, when translated, lead to proteins differing from one another in two or more amino acid sequence positions. By choosing the nucleobase sequence of compatible ends of fragments, it is thus possible to maintain a defined chain relationship of fragments while allowing combinations of different fragment species, i.e. fragments differing from each other only in few positions.

In addition, it is possible to introduce point mutations other than in the sections corresponding to a primer used in step a) of the alteration method. For example, by using nucleotides with non-standard nucleobases during amplification step b) or by chemical modification after amplification step b), additional mutations can be introduced.

One use of the joining method of the present invention also allows to create a joined nucleic acid product differing from a template by the order of sequence sections. For example, primers may be chosen to have such nucleobase sequences that by hybridization under stringent conditions the order of corresponding fragments is reversed or otherwise differs from the order of corresponding sections of the template nucleic acid. By the same way, the invention allows to produce joined nucleic acids differing from a template by the orientation of sequence sections.

The joined nucleic acid product may also differ from a template by the number of sequence sections. For example, the compatible ends of fragments can be chosen such that sections are joined which in the template ore reference nucleic acid are separated from each other by an intermittent section. Such alteration is for example preferred for deletion of introns. Also, additional segments can be included between sections which, in the template nucleic acid, are adjacent to one another. And the joined nucleic acid product may differ from a template by comprising repetitions of sections. The number of section repetitions can be controlled by appropriately choosing the nucleobase sequence of compatible ends such as to maintain a chain relationship. On the other hand, a random number of section repetitions can be achieved by choosing the respective compatible ends of segments such that a segment (or the intermediate product of joining two or more fragments) is compatible to itself, allowing a repetition of segments ad infinitum.

Also as indicated above, the alteration and joining method of the present invention can be used to create an operative linkage of a nucleic acid segment to a transcription and/or translation influencing site. Such method is preferred for example when it is desired to control expression of a selected gene by a transcription and/or translation regulation mechanism such that the selected gene can be switched on and/or off in a transformed cell under preselected conditions. One preferred way is to bring one or more selected gene(s) under the control of a lac repressor element to selectively allow transcription and translation of the selected gene upon exposure of the transformed cell to IPTG. Also, ribosome binding sites or other operator binding elements could be attached to a nucleic acid or gene as desired, for example to introduce a protein tag (e.g. His-Tag, myc-Tag, HA-Tag, FLAG-Tag, TAP-Tag), protein fusion linker (e.g. Ala₍ₙ₎ linker) or signal sequences for the cellular localization. Further possible modifications are the introduction of introns in a coding nucleic acid product. Additional transcription and/or translation influencing sites which can according to the present invention be (a) operatively linked to a selected nucleic acid fragment or (b) altered are activator or repressor binding sites, 5'-UTR regions, riboswitches and terminator regions,

The alteration and/or joining method of the present invention can also be used to create a joined nucleic acid product which differs from a template or reference nucleic acid by the order and/or number and/or length of transcribed and/or translated sequence sections. The advantages and means to use the method of the invention accordingly have been described above.

By combining nucleic acid segments according to the present invention it is possible to create new or altered nucleic acid sequences for transcription and/or translation. The present invention thus offers a surprisingly fast, reliable and not laborious way to create new proteins (e.g. of different stability with regards to temperature, pH, proteases and/or organic solvents, or different physiological properties, e.g. substrate specificity and/or turnover, inhibitor specificity and/or sensitivity, activator specificity and/or sensitivity, or different cellular transport properties, e.g. by comprising cellular localization signals, or purification properties, e.g. by comprising His tags or other tags as indicated above) and nucleic acids (e.g. antisense RNAs), using for example any of the above means. In particular, the alteration and joining method of the present invention allows to modify one or more of:
The nucleotide sequence of one or more exons,
The nucleotide sequence of one or more introns,
The nucleotide sequence of such coding region (for example to optimize the sequence in accordance to codon preferences of a target organism), the number and/or order of exons, deletion of one or more introns, replacement of one or more introns by one or more other introns,
The distance of coding regions on a nucleic acid, for example by fusion of two or more previously separately transcribed coding regions to create a chimerical protein,
The organization of a nucleic acid, for example for creating a transcribed nucleic acid comprising two or more coding nucleic acid segments to generate an operon,
The regulation of nucleic acid, for example the location and/or sequence of a promoter or other transcription factor binding site, and/or the location and/or sequence of a transcription terminator, for example to create an expression cassette by operatively linking a coding nucleic acid segment to a promoter and/or terminator element, wherein the promoter can be constitutively active, inducible or repressible to regulate transcription and/or translation of an operon, wherein the expression cassette can comprise further transcription and/or translation influencing elements,
Other functions of nucleic acids segments, for example the location and/or sequence of matrix or scaffold attachment sites, restriction endonuclease binding sites, origins of replication, packaging signal sequences,

The amino acid sequence of one or more proteins, for example by adding, modifying or removing proteolytic enzyme cleaving sites, cellular localization signals, glycosylation sites, domains and folding structures.

Such modifications can be made according to the present invention (a) in a defined way by linking nucleic acid fragments in a defined order, wherein optionally one or more nucleic acid fragments have been created based on one or more template nucleic acids as described above, and (b) in a randomized way by more or less randomly linking of such nucleic acid fragments to create a library of different nucleic acids, as will be described in more details below with respect to the recombination and/or shuffling method of the present invention. The different nucleic acids can comprise, for example, isoforms or homologous forms of coding nucleic acid segments, e.g. of different cell types or organisms. Thus the present invention also allows to produce a library of gene clusters for metabolic pathways.

The methods of the present invention are particularly suitable for nucleic acid recombination techniques, particularly shuffling techniques, and specifically DNA shuffling. Generally, such techniques involve the steps of enzymatically fragmenting a template nucleic acid to obtain nucleic acid fragments, amplification of single-stranded nucleic acid fragments by a nucleic acid polymerase, normally a DNA polymerase, according to the overlap extension PCR scheme, and finally amplification of the complete nucleic acid for inserting of a single insert into a vector. The present disclosure avoids several drawbacks of this technique by providing a nucleic acid recombination method comprising the steps of
a) selecting a set of nucleic acid sections and primers for amplification of said sections, wherein the primers comprise nucleotides configured for non-enzymatic removal as described above,
b) amplification of said sections to obtain a set of corresponding nucleic acid fragments comprising nucleotides configured for non-enzymatic removal ,
c) joining of fragments obtained in step b), and optionally a vector fragment, by a joining method of the present invention,
d) transforming the joined nucleic acid obtained in step c), preferably as described below.

The recombination or shuffling method particularly abolishes the need for a final nucleic acid amplification or ligation of joined nucleic acid fragments before transformation. Also, the fragments can be prepared individually and do not have to be prepared ad hoc by restriction nuclease treatment. Thus, it is particularly easy to obtain defined quantities of desired nucleic acid fragments without losses as would be required for isolation of desired fragments from electrophoresis gels. Another advantage of the present invention is, as described above, that by appropriate selection of the primers, which provide the compatible ends in the joining method of the present invention, it is possible to restrict the number of combinations possible for the selected set of fragments. Also, as the length of compatible ends can be rather short, particularly 10 to 18 nucleotides and most preferably 11 to 14 nucleotides, the possible range of fragment combinations is nearly not limited for any practical purpose, particularly since the compatible ends may comprise one, two or more base mismatches under hybridizing conditions.

The nucleic acid recombination or shuffling method of the present disclosure, preferably in the form of a DNA shuffling method of the present invention, is particularly useful for recombination or shuffling of exons, conserved sections of nucleic acids, protein domains, nucleic acid domains like hairpin loops, regulatory elements, homeodomains and homologous genes. The recombination or shuffling method is almost independent of homologies and, by any practical definition of the term, not limited by the number of fragments to be recombined or shuffled. The method allows to generate large libraries with a low fraction of unshuffled wild type sequences.

The nucleic acid recombination or shuffling method of the present disclosure could also be used for the recombination or shuffling of whole gene clusters or pathways consisting of more than one gene or expression units. Expression units consist of a coding region which is operatively linked to one or more regulatory sequences.

Also as indicated above, it is a particular advantage that the joining method and, optionally, the alteration method of the present invention can be used to prepare joined nucleic acids for transformation purposes. Thus according to the invention there is also provided a transformation method, comprising the steps of
a) performing a nucleic acid joining method as described above, optionally also comprising the steps of a nucleic acid alteration method of the present invention, and
b) transforming a target cell with the joined nucleic acid obtained in step a).

As described above, it is a particular advantage of the present invention that the joined nucleic acid obtained in step c) of a joining method of the present invention can be used directly for transformation of a target cell without the need to perform a prior nucleic acid amplification or ligation step. The method of the present invention thereby obviates two steps which could introduce artifacts or lower the transformation efficiency. Thus, a transformation method of the present invention is particularly useful for creating a large number of transformants per mol nucleic acid. The present disclosure thereby provides a method for creating a cloning library, comprising the steps of:
a) performing a nucleic acid alteration method of the present invention to obtain a set of different nucleic acids, and
b) transforming a set of target cells with the nucleic acids obtained in step a).

The term "transformation" or "transforming" according to the invention is not limited to the alteration of genetic material of prokaryotic cells. Instead, the terms denote the introduction of nucleic acid into any kind of cell, i.e. a closed compartment capable of performing replication of nucleic acids, and thereby also includes methods also known as transduction and transfection. Thus, the transformation method of the present invention allows to introduce nucleic acids into prokaryotic cells and into eukaryotic cells. Preferred cells are selected from those of the group consisting of *Escherichia coli, Bacillus subtilis,* Pseudomonas, Xanthomonas, Corynebacterium, Hansenula, Leishmania, Streptomyces, Mycobacterium, Agrobacterium, *Saccharomyces cerevisiae, Pichia pastoris, Schizosaccharomyces pompe,* Penicillum, Aspergillus, Trichoderma and Chinese Hamster Ovary cells.

Transformation is preferably achieved via electroporation, calcium phosphate precipitation, heat shock transformation, protoplast transformation (e.g. Chang et al., Molec. gen. Genet. 1979(168), 111-115), lipofection, particle based transformation (for example GeneGun), transfection, transduction.

The transformation method of the present invention using one or more nucleic acids joined according to the joining method of the present invention advantageously achieves high transformation efficiency as measured by number of transformant colonies per µg nucleic acid, preferably DNA. As will be described in the examples, high transformation efficiency can be achieved by joining a commercial vector of 5-6 kb and 5 insert fragments of 0.1-0.5kb. Transformation efficiency was so high that even a library of shuffled insert fragments can be prepared easily.

For transformation purposes, it is preferred that one of the fragments used in the joining method of the present invention forms a vector, or that two or more fragments combine to form a vector. As indicated above, a vector according to the invention is a nucleic acid which allows replication of said nucleic acid in the target cell. The joined nucleic acid thus comprises the vector and one or more additional "insert" nucleic acid(s).

The joining method of the present invention can easily be performed using a kit of the invention for joining at least three nucleic acid fragments, said kit comprising
- for each fragment, two amplification primers having nucleotides configured for non-enzymatic removal, and
- one or more non-enzymatic nucleotide removal agents, preferably iodine, ethanol or a mixture thereof.

Also according to the present disclosure, there is provided a computer program product, comprising a machine interpretable data carrier, said data carrier comprising a set of instructions which, when interpreted by a nucleic acid handling apparatus, cause said apparatus to perform a method of the present disclosure. Particularly, the instructions can cause the nucleic acid handling apparatus to dispense at a selected time iodine and ethanol to one or more vessels comprising nucleic acids, wherein the nucleic acids comprise nucleotides comprising phosphorothioate groups, and/or to amplify nucleic acids by performing a heating and cooling protocol useful in PCR methods.

### EXAMPLES

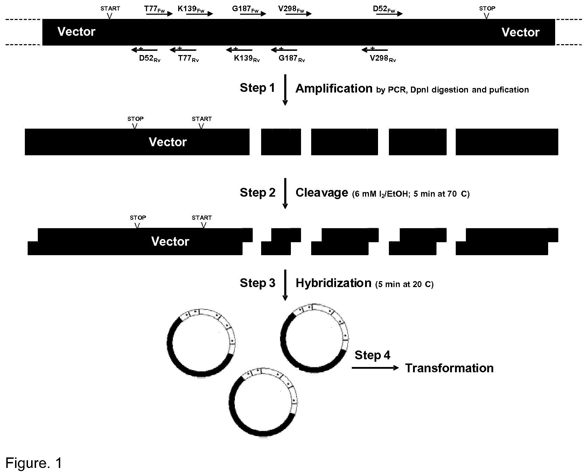

The invention is hereinafter further described by means of figures and examples to highlight selected aspects of the invention. The examples shall not be interpreted to limit the scope of the claims or of other sections of the detailed description.
Example 1: "OmniChange" sequence independent method for simultaneous site saturation of five codons
Figure 1: OmniChange comprises four steps: Step 1. Amplification of five DNA fragments bearing a NNK-saturated codon (indicated with *). Step 2. Chemical cleavage to generate complementary single-stranded 5'-overhangs. Step 3. Hybridization of all fragments to a full circular plasmid containing 10 DNA nicks. Step 4. Transformation and nick-repair in *E*. *coli* BL21-Gold (DE3) Iacl^{Q1}

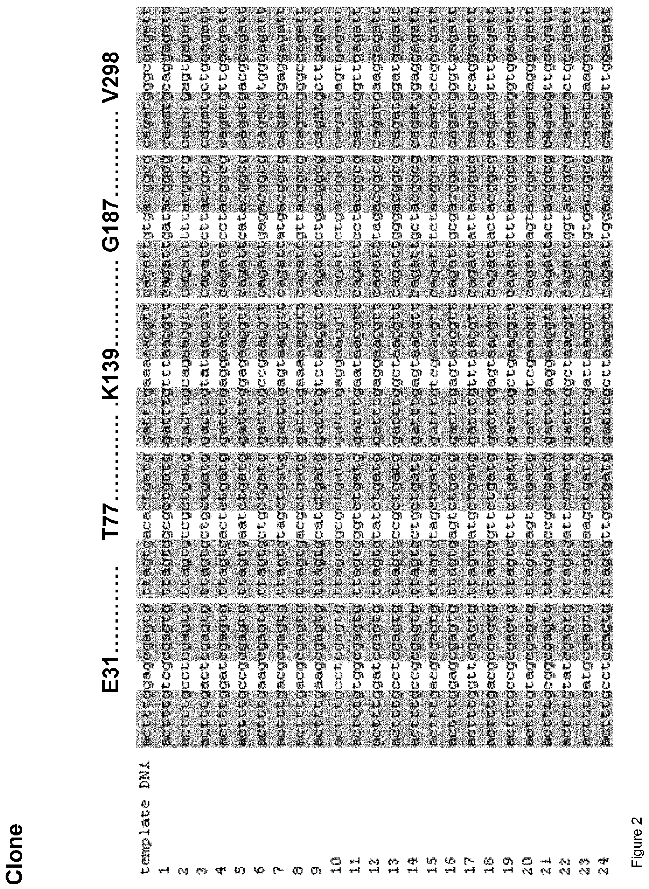

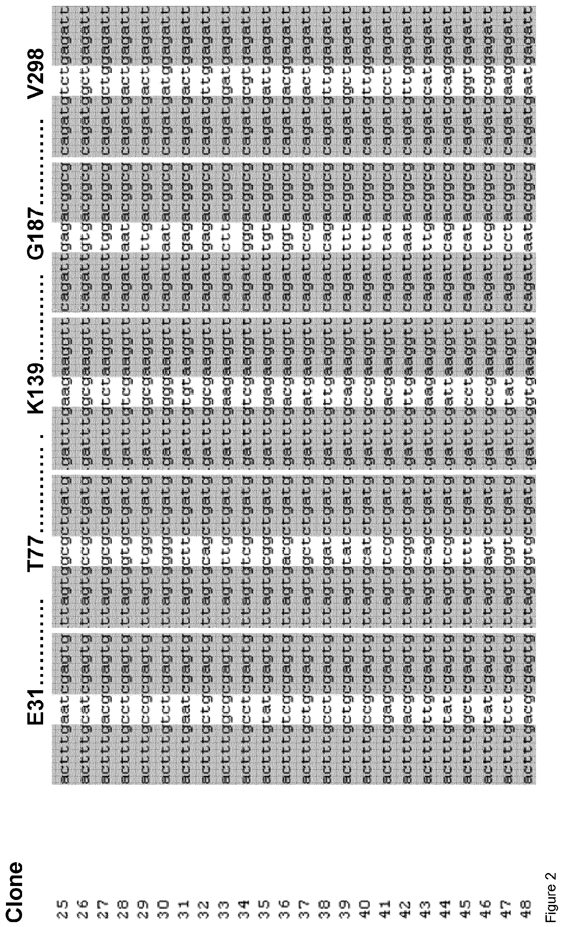

Figure 2: Partial sequence alignment of wild-type gene (template DNA) to 48 random clones from a multi site-saturation library generated by OmniChange. The five sites targeted for saturation are highlighted in white. Arrows indicate the distance between the targeted positions in base pairs. A comparison of the codons in the white columns shows obtained diversity by saturating five independent positions simultaneously by OmniChange.

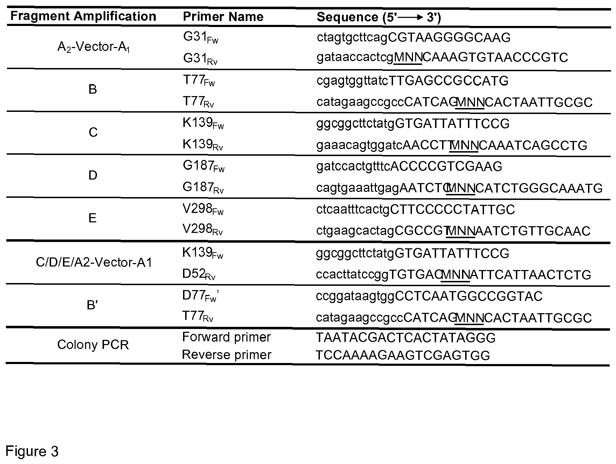

Figure 3: Oligonucleotides used in the development of OmniChange. Lower case letters indicate phosphorothioated bonds and underlined nucleotides in the reverse primers highlight the NNK degenerated codons.

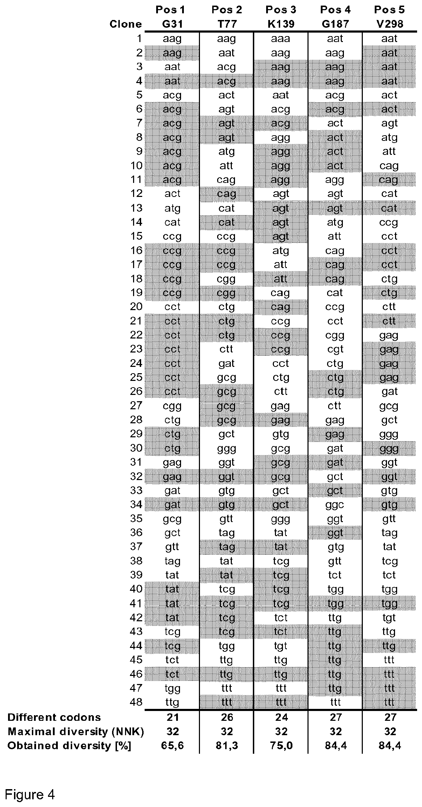

Figure 4: Overview on codon diversity of five focused NNK-randomized positions from 48 randomly picked clones after an OmniChange experiment. Grey highlighted codons occur more than once at the respective position.

### Enzymes, reagents and oligonucleotides

All reagents used were of analytical grade and purchased from Sigma-Aldrich (Steinheim, Germany) and AppliChem (Darmstadt, Germany). Enzymes were obtained from New England Biolabs (Frankfurt, Germany), and dNTPs were purchased from Fermentas (St.Leon-Rot, Germany). Oligonucleotides were synthesized at HPLC-purity by Eurofins MWG Operon (Ebersberg, Germany) in salt-free form. According to suppliers recommendations all oligonucleotides were diluted in Milli-Q water to a final concentration of 100 µM. All oligonucleotides used in this study are summarized in Figure 3.

### Cell strains and vectors

The 1.3 kb phytase (phosphatase) gene from *Yersinia mollaretii* (81 % sequence identity with phytase gene from *Yersinia intermedia* (BLAST); GenBank: DQ986462.1) was cloned and expressed in a pET-22b(+)-derived Novagen vector (Darmstadt, Germany) named pALXtreme-5b (2.1 kb) (Blanusa, M. et al., (2010) Phosphorothioate-based ligase-independent gene cloning (PLICing): An enzyme-free and sequence-independent cloning method. Anal. Biochem., 406, 141-146). Gene expression from pALXtreme-5b requires *Escherichia coli* BL21-Gold (DE3) Iacl^{Q1} as host system due to necessary incorporation of the lacl repressor gene into the bacterial genome (Blanusa, M. et al., (2010) Phosphorothioate-based ligase-independent gene cloning (PLICing): An enzyme-free and sequence-independent cloning method. Anal. Biochem., 406, 141-146). Chemically competent *E*. *coli* BL21-Gold (DE3) Iacl^{Q1} cells were prepared according to a standard protocol (Inoue,H. et al., (1990) High efficiency transformation of Escherichia coli with plasmids. Gene, 96, 23-28) with a determined transformation efficiency of 1.4 x 10⁶ cfu/µg pUC19.

### Mutagenic oligonucleotide design

Ten oligonucleotides were designed as PCR-primers for simultaneously saturating five codons (Figure 3). Reverse primers harboring a NNK codon were designed according to Stratagene's QuikChange Site-Directed Mutagenesis Kit Manual (La Jolla, CA, USA) for optimization of PCR product yields. In addition, all oligonucleotides have at least 12 phosphorothioate nucleotide bonds at the 5'-terminus to generate single stranded overhangs for DNA hybridization after iodine cleavage (Blanusa, M. et al., (2010) Phosphorothioate-based ligase-independent gene cloning (PLICing): An enzyme-free and sequence-independent cloning method. Anal. Biochem., 406, 141-146) (Figure 3).

### PCR amplification of DNA fragments

Standard plasmid isolation was accomplished by using the QIAGEN QIAprep Spin Miniprep Kit (Hilden, Germany). A standard PCR set up contained 1x Phusion DNA polymerase buffer, 0.2 mM dNTP mix, 0.4 µM fragment specific forward and reverse primer (Figure 3), 5 U Phusion DNA polymerase and 20 ng of DNA template in a final volume of 50 µl. All PCR amplifications were carried out in an Eppendorf Mastercycler proS (Hamburg, Germany) using thin-wall PCR tubes (Sarstedt, Nuembrecht, Germany). A PCR for targeted codons was performed by pre-heating each fragment sample mixture to 94°C for 3 min followed by 25 cycles of 94°C for 30 s, 55°C for 30 s and 72°C for 30 s (fragments B, C, D and E) or 1.5 min (fragment A₂-Vector-A₁). For final elongation a fill-up cycle was performed at 72°C for 1 min (fragment B, C, D and E) or 3 min (fragment A₂ Vector-A₁). PCR products were separated on 2 % (w/v) Tris-acetate-EDTA (TAE) agarose gels to confirm the size of the respective PCR products (Maniatis,T. et al., (1982) Molecular Cloning: A Laboratory Manual. Cold Spring Harbor Laboratory Press, Cold Spring Harbor, N.Y., 1-545). Specific degradation of methylated template DNA was realized by adding 1 µl (20 U) of Dpnl to each PCR sample. After incubation (37°C for 4 h) the PCR products were purified using the Macherey-Nagel NucleoSpin Extractll DNA purification kit (Dueren, Germany) and eluted in 50 µl Milli-Q water. Purified PCR products were quantified using a Nanodrop 1000 UV spectrophotometer (NanoDrop Technologies, DE, Wilmington, USA).

### Iodine cleavage, DNA-fragment annealing and transformation

All steps were performed on ice unless stated otherwise. Purified fragment A₂₋Vector-A₁ and fragments B, C, D, and E were diluted to 0.02 pmol/µl or 0.11 pmol/µl respectively using Milli-Q water. The PCR-products were cleaved according to the PLICing cloning technology (Blanusa, M. et al., (2010) Phosphorothioate-based ligase-independent gene cloning (PLICing): An enzyme-free and sequence-independent cloning method. Anal. Biochem., 406, 141-146) with the following alterations: 4 µl of each PCR fragment were combined with 2 µl iodine cleavage buffer (0.25 M Tris-HCl pH 9.0; 30 mM iodine; 30 % (v/v) ethanol) in PCR tubes, incubated in a thermocycler (70°C; 5 min) and finally kept on ice (5 min). Annealing of cleaved fragments was performed gradually by first adding 6 µl fragment B cleavage mix to 6 µl of fragment A₂₋Vector-A₁ cleavage mix. Hybridization of both fragments was performed at 20°C for 5 min. Subsequently, cleavage mixtures of fragments C, D and E were added and hybridized, yielding a circular and nicked B-C-D-E-A₂₋Vector-A₁ construct. The mixture was kept on ice for further 5 min. Self-hybridization of the A₂₋Vector-A₁ fragment was determined by preparing solely the A₂₋Vector-A₁ fragment identically to the nicked B-C-D-E-A₂-Vector-A₁ construct. In the latter quality control, fragments B, C, D and E were replaced each by 4 µl Milli-Q water. Finally, 4 µl of both hybridization mixtures were transformed via heat shock and without any further purification step into 100 µl chemical competent *E*. *coli* BL21-Gold (DE3) Iacl^{Q1} cells (Hanahan,D.et al., (1991) Plasmid transformation of Escherichia coli and other bacteria. Methods Enzymol., 204, 63-113). Transformation mixtures were spread on agar plates (LB; 100 µg/ml ampicillin) and incubated overnight.

### Colony PCR and sequencing of plasmid constructs

Colony PCRs were performed to verify the correct assembly of five fragments to the B-C-D-E-A₂-Vector-A₁ construct (Figure 1) carrying five saturated codons. Twenty colonies were randomly picked into liquid LB media (2 ml, 100 µg/ml ampicillin) and grew overnight (14 h, 37°C, 250 rpm). Liquid cultures (10 µl) were lysed (50 µl Milli-Q water, 95°C, 15 min), centrifuged (13000 g, 2 min) and 5 µl of clear supernatant served as template DNA for colony PCR-amplifications (1x Taq DNA polymerase buffer, 0.2 mM dNTP mix, 0.4 µM Forward and Reverse primer (Figure 3), 5 U *Taq* DNA polymerase; identical cycling program as for fragment A₂₋Vector-A₁ amplification). Elongation times in each PCR cycle were increased to 1 min and to 3 min in the fill up reaction. PCR samples were separated on 1 % (w/v) TAE agarose gels to verify the size of each PCR product. Sequencing was performed at GATC Biotech (Konstanz, Germany) and sequence analysis was carried out using Clone Manager 9 Professional Edition software (Scientific & Educational Software, Cary, NC, USA).

### Phytase activity measurements in microtiter plates

For phytase activity measurements, randomly picked colonies were transferred from agar plates into a Greiner Bio-One 96-well microtiterplate (Greiner, Frickenhausen, Germany) filled with 200 µl sodium acetate (NaAc) buffer (pH 5.5, 250 mM sodium acetate; 1 mM CaCl₂; 0.01 % (w/v) Tween20). Enzymatic reactions were initiated by adding 50 µl NaAc buffer containing 4-methylumbelliferylphosphate (1 mM). Phytase activity was determined by following the fluorescence increase due to 4-methylumbelliferone formation (Aₑₓ: 360 nm; λₑₘ: 460 nm) (Garrett,J.B.et al., (2004) Enhancing the thermal tolerance and gastric performance of a microbial phytase for use as a phosphate-mobilizing mo) using an Infinite M 1000 plate reader (Tecan, Groeding, Austria, 37°C for 15 min). Colonies with the original (template) phytase gene and colonies only carrying the empty pALXtreme-5b vector were used as reference.

### Results

The OmniChange multi site-saturation method consists of four steps: Step 1. Fragment generation by PCR with oligonucleotides containing NNK-codons, Step 2. DNA-cleavage reaction for the generation of complementary 5'-overhangs, Step 3. Assembly via DNA-hybridization and Step 4. Transformation and nick repair by *E. coli* BL21-Gold (DE3) Iacl^{Q1} (Figure 1).

Amino acid positions G31, T77, K139, G187 and V298 of a phytase from *Yersinia mollaretii* were selected for NNK-saturation. The latter five codons were identified in a directed phytase evolution experiment using the sequence saturation mutagenesis method (Wong,T.S. et al., (2004) Sequence saturation mutagenesis (SeSaM): a novel method for directed evolution. Nucleic Acids Res., 32, e26) for random diversity generation and being screened for improved thermal resistance (unpublished results).

Oligonucleotides for the fragment generation (Step 1) can be designed with the general sequence scheme 5'-(nt)*₁₂-NNK-(nt)₁₂-3'. Further optimizations for oligonucleotide design (Tₘ and GC content) were performed according to the QuikChange Site-Directed Mutagenesis Kit manual (Stratagene, La Jolla, CA, USA). The twelve nucleotides on 5'end (nt*) are connected through a phosphorothiodiester bond (Eckstein,F. et al., (1989) Phosphorothioates in molecular biology. Trends Biochem. Sci., 14, 97-100) in contrast to the residual minimal recommended twelve nucleotides on 3'end and NNK codon in the respective reverse primers that are connected through natural phosphodiester bonds (Figure 3). Successful PCR-amplification of the five DNA-fragments A₂-Vector-A₁, B, C, D and E (Step 1) was verified by agarose gel electrophoresis (A₂-Vector-A₁: 2571 bp; B: 160 bp; C: 199 bp; D: 157 bp and E: 345 bp). After Dpnl digestion, iodine cleavage (Step 2), hybridization (Step 3) and transformation into *E*. *coli* BL21-Gold (DE3) Iacl^{Q1} (Step 4) 392 colonies were obtained on a single agar plate assuming a transformation efficiency of 10⁴ cfu/µg for five DNA fragments (transformation efficiency of self-made competent cells: 1.4 x 10⁶ cfu/µg pUC19). Various optimization steps in fragment preparation, cleavage and hybridization were performed until a sufficient amount of colonies (> 1000 colonies per µg hybridized DNA) could be obtained.

The following "control experiments" were performed in order to access the versatility and robustness of the developed OmniChange mutagenesis method. First a possible vector fragment self-hybridization (DNA-fragment A₂-Vector-A₁) was determined, yielding four colonies that represent ∼1 % of the overall variant number under identical conditions. Secondly, nick repair and assembly of full length plasmid and genes was verified by DNA-restriction analysis and colony PCR of 20 randomly picked clones showing that complete template DNA information could be assembled by OmniChange with 100 % efficiency. Furthermore, five randomly picked clones were sequenced and all five randomized positions were unique differing from the corresponding wild-type sequence. As last control the phytase activity of 20 randomly picked colonies was determined using a 4-methylumbelliferylphosphate detection system (Garrett,J.B.et al., (2004) Enhancing the thermal tolerance and gastric performance of a microbial phytase for use as a phosphate-mobilizing mo). Surprisingly all twenty variants, despite of the high mutational load, showed at least a low but detectable activity proving functional expression from assembled plasmids. Latter controls prove that up to ten nicks can be ligated by *E. coli* BL21-Gold (DE3) Iacl^{Q1} and generated complementary ends (12 nt overhangs) can withstand transformation conditions.

For detailed statistical analysis, 48 randomly picked clones were sent for forward and reverse sequencing. Multi-sequence alignments with the wild-type sequence confirmed that all five codons were fully saturated and no clone retained the original sequence (Figure 2 & Figure 4). Of the 48 sequences analyzed, one wild-type codon could be found at amino acid positions G31 and G187, respectively two wild-type codons on position V298. At amino acid position T77 not a single wild-type codon could be identified due to the fact that the NNK does not offer the respective wild-type codon (Firth,A.E. et al., (2008) GLUE-IT and PEDEL-AA: new programmes for analyzing protein diversity in randomized libraries. Nucleic Acids Res., 36, W281-285). Surprisingly, on position K139 one wild-type codon was identified (clone 8) after sequencing although NNK does not offer AAA as randomly introduced codon (Figure 2). For this case we have to assume that either oligonucleotide synthesis was not performed completely error-free or exonuclease activity of Phusion polymerase corrected the mismatches during PCR. NNK degeneracy offers a diversity of 32 different codons at each position covering all canonical amino acids meanwhile avoiding two of three stop codons (Reetz,M.T. et al., (2008) Addressing the numbers problem in directed evolution. ChemBioChem, 9, 1797-1804). Twenty-one different codons at position G31 could be observed, resembling already 65.6 % of the possible diversity after sequencing of only 48 clones. For all other positions even a higher coverage could be obtained: T77 (26 unique codons 81.25 %), K139 (24 unique codons 75 %), G187 (27 unique codons 84,4 %), V298 (27 unique codons 84.4 % diversity) (Figure 4). Site-specific cleavage of phosphorothioated nucleotides from 5'-ends of double-stranded DNA was developed as cloning technology reporting that the cleavage reaction did not cause any additional mutations (Blanusa, M. et al., (2010) Phosphorothioate-based ligase-independent gene cloning (PLICing): An enzyme-free and sequence-independent cloning method. Anal. Biochem., 406, 141-146). Sequence analysis of the 48 clones also showed that the cleavage reaction (Step 2) and fragment hybridization (Step 3) did not introduce additional point mutations nor caused insertions or deletions.

Furthermore, OmniChange allows a modular and combinatorial assembly of the targeted codons to study additive and cooperative effects in detail and for any target position combination. With the same sets of oligonucleotides position D52 was selected to investigate whether codons with less than 100 bp distance to each other could be targeted. As proof of principle, two libraries were generated: library 1 targeting positions D52 and T77 (primer combinations K139_{Fw}/D52_{Rv}, (fragment C-D-E-A₂-Vector-A₁) and T77_{Fw}7T77_{Rv} (fragment B'); see Figure 3) and library 2 targeting T77, K139, G187 and V298 (oligonucleotide combinations in Table1 yielding fragments C, D, E and A₂-Vector-A₁-B). Sequencing of five randomly picked clones from both libraries showed again that targeted codons were saturated exclusively whereas all other positions remained unchanged (data not shown).
Example 2: An enzyme-free and sequence independent method for the combinatorial assembly of multiple DNA fragments
Figure 5: Scheme of Domain-PLICing comprising four steps: 1) Amplification of individual domains, 2) Chemical cleavage to generate single-stranded overhangs at cross-over-sites, 3) One-pot recombination and hybridization of all DNA-fragments and 4) Transformation.
Figure 6: Shuffling results of 42 randomly picked clones by Domain-PLICing. Identity of each domain was verified by DNA-sequencing. All clones are arranged in the order of sequencing and the origin of each domain is highlighted by color: white: phyY; grey: appA; black: phyX.
Figure 7: Oligonucleotides used in the development of Domain-PLICing. Lower-case letters indicate phosphorothioated bonds and nucleotides, necessary to mutate for complementary 5'-overhangs, are underlined.

### Enzymes, reagents and oligonucleotides

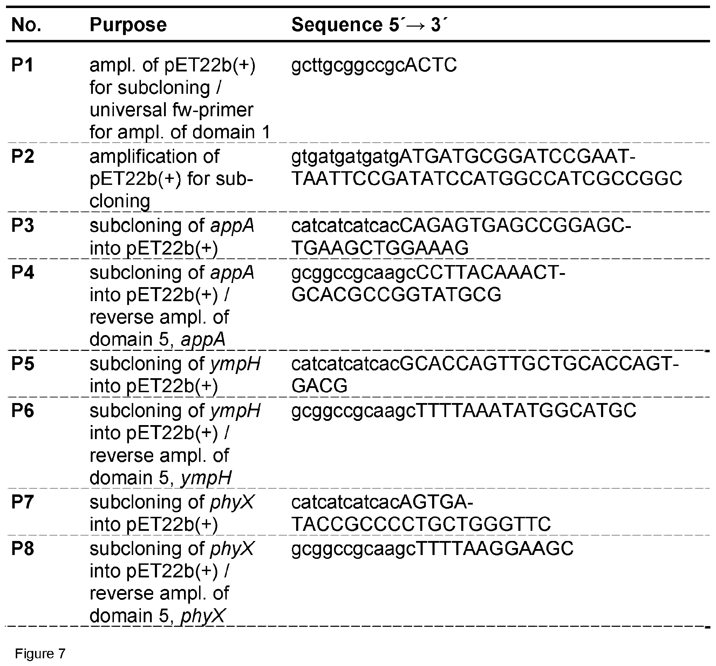

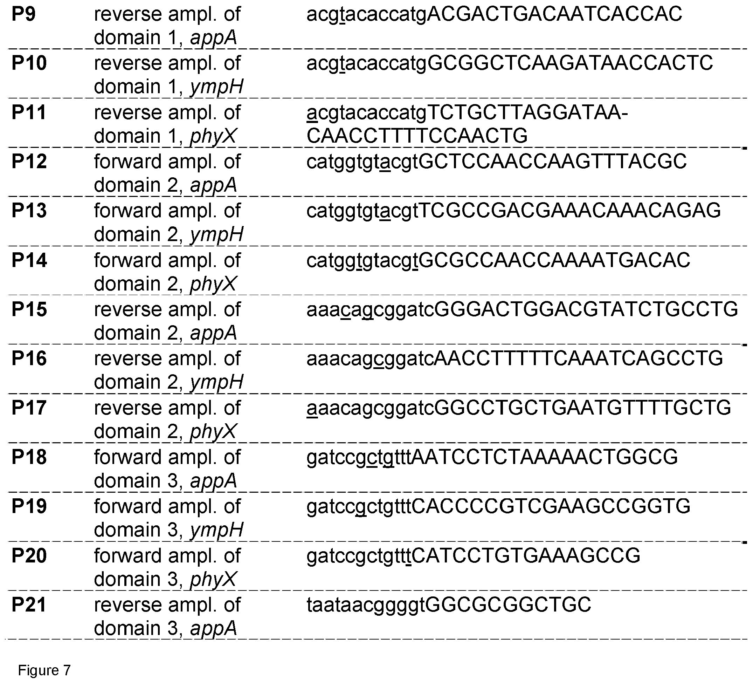

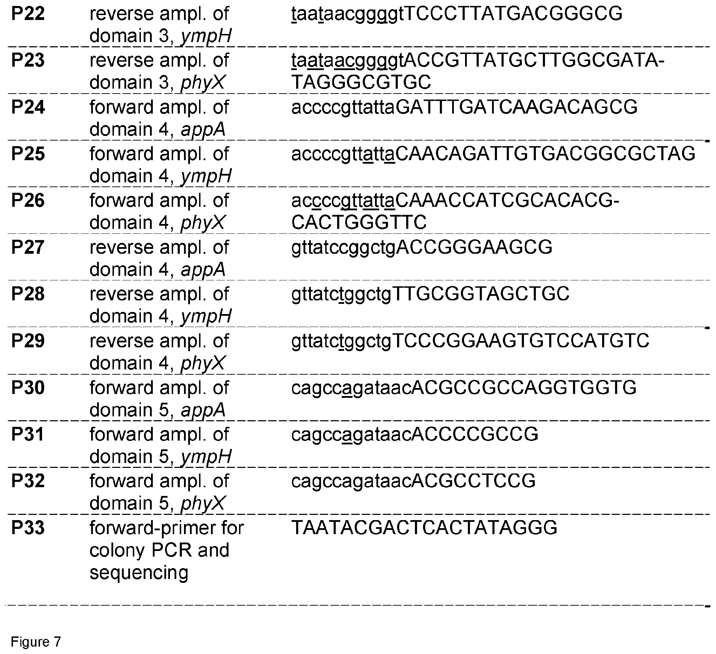

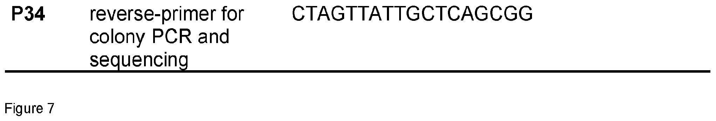

All chemicals were purchased from Sigma-Aldrich (Steinheim, Germany), Serva (Heidelberg, Germany) or AppliChem (Darmstadt, Germany) and were analytical grade unless stated otherwise. Enzymes and corresponding buffers were obtained from Fermentas (St. Leon-Rot, Germany) and all oligonucleotides were HPLC-purified and purchased from Eurofins MWG Operon (Ebersberg, Germany) in salt-free form. Oligonucleotides were diluted in Milli-Q water to a final concentration of 100 µM. All oligonucleotides used in this study are summarized in Figure 7.

### Strains, genes and vectors

The genes of the three phytases *appA* of *Escherichia coli* (GenBank: AF537219), phyY of *Yersinia mollaretii* (GeneBank: NZ_AALD02000002) and phyX of *Hafnia sp.* LU11047 (WO2011048046, SeqID 12) were ordered as synthetic genes (GENEART AG, Regensburg). The expression vector pET22b(+) was obtained from Novagen (Merck KGaA, Darmstadt, Germany) and *E*. *coli* BL21-Gold (DE3) was purchased from Invitrogen (Karlsruhe, Germany). Chemically competent *E*. *coli* BL21-Gold (DE3) cells, with a determined transformation efficiency of 1.4 x 10⁶ cfu/µg pUC19, were prepared according to a published procedure (Inoue et al., 1990).

### PCR amplification of phytase genes, individual phytase domains and vector pET22b(+)

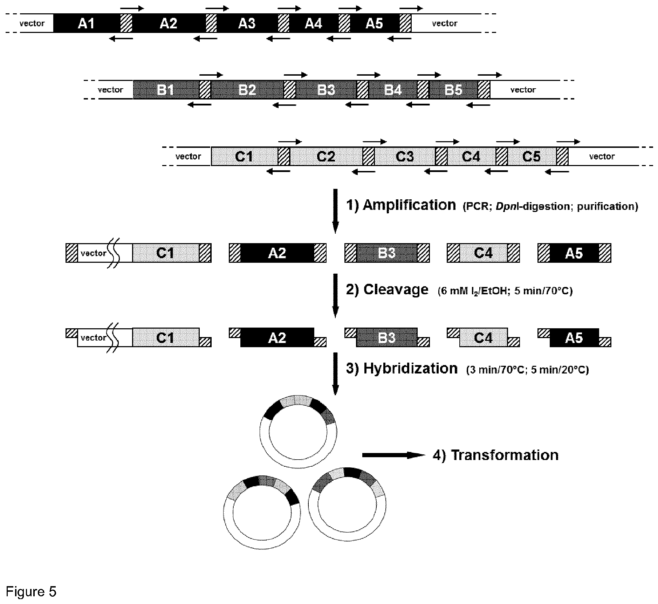

All DNA-isolations were performed using QIAGEN QIAprep Spin Miniprep Kit (Hilden, Germany). Domain-PLICing is almost sequence independent, since it requires only a short stretch of four identical amino acids as crossover point for recombining DNA fragments. In the three model phytases four crossover points were identified by multiple alignments, giving rise to five different protein domains varying in size (Domain A: -50 AS; Domain B: -105 AS; Domain C: -155 AS; Domain D: -50 AS and Domain E: -55 AS). Although being identical on the protein level, did the crossover points show diversity on the gene level due to the degeneracy of the genetic code. Therefore, in total 35 silent mutations were introduced into 19 out of 30 primers during oligonucleotide design for PCR-amplification of all domains, since Domain-PLICing also demands sequence identity on the gene level at respective crossover points (see Figure 7). All PCRs were performed in a final volume of 50 µl in an Eppendorf Gradient Cycler (Darmstadt, Germany) using thin-wall PCR tubes (Sarstedt, Nuembrecht, Germany). PCR-setups for subcloning purposes were composed of 1x *PfuS* buffer, 0.2 mM dNTP mix, 400 nM forward and reverse primer (*appA*: P1 and P2; *phyY*: P3 and P4; *phyX*: P5 and P6; see Figure 7), 5 U *PfuS* DNA polymerase and 20 ng template-DNA. Cycling started with an initial denaturation at 94°C for 2 minutes. Subsequently, 35 cycles (94°C, 30 s; 55°C, 35 s; 68°C for 50 s (phytases) or 4 minutes (pET22b(+)) and one fill up cycle (68°C; 10 min) were performed. Before iodine-cleavage, PCR-products were DpnI-digested to remove template-DNA, purified using the PCR purification kit (Macherey-Nagel, Dueren, Germany) according to manufacturer's instructions and quantified using a NanoDrop (NanoDrop Technologies, Wilmington, DE, USA). For amplification of individual phytase-domains, PCR-setups contained 1x *Taq* buffer, 0.2 mM dNTP mix, 400 nM forward and reverse primers (see Figure 7), 5 U Taq DNA polymerase and 40 ng of the respective pET22b(+)-vector templates. Cycling started with an initial denaturation at 94°C for 2 minutes. Subsequently performed were: 35 cycles (94°C, 30 s; 55°C, 30 s; 72°C for 7 minutes (domains A1, B1 and C1, which include the pET22b(+)-vector sequence; see Figure 5) or 1 min (all domains 2 - 5; see Figure 5) and one fill up cycle (72°C; 5 min). Resulting PCR-products were first *Dpn*I-digested to remove template-DNA, subsequently purified and finally quantified (NanoDrop). DNA-samples were aliquoted to 5 µl and stored at -20°C until further use. Agarose-TAE gel electrophoresis was performed according to a standard protocol (Maniatis et al., 1982) to confirm presence and size of amplified genes, individual domains or vectors. Colony PCRs were performed before sequencing, following the subcloning-PCR protocol with only slight modifications: A master mix was prepared and dispensed into 20 µl aliquots in PCR tubes. Colonies were directly transferred from a master plate into PCR tubes with a sterile pipette tip. Initial denaturation time at 94°C was increased to 5 min to ensure cell-lysis, and 5 µl of the resulting PCR-products were subsequently analyzed by agarose gel-electrophoresis for DNA fragment size determination.

### Iodine treatment, DNA-fragment hybridization and transformation

For recombination purposes, iodine treatment and DNA-fragment hybridization procedures were optimized to recombine multiple DNA-fragments using the phosphorothioate cleavage principle reported by Blanusa et al., 2010. Finally, a mixture of 4 µl DNA-sample (0.03 - 0.6 pmol), 0.5 µl cleavage buffer (0.5 M Tris-HCl pH 9.0), 0.3 µl iodine stock solution (100 mM iodine in 99 % ethanol) and 0.2 µl of Milli-Q water proved to be best for DNA-cleavage. Samples were incubated in PCR tubes (70°C; 5 min; Eppendorf Mastercycler) and kept on ice until further use. Generated DNA-fragments were subsequently hybridized without any further purification by carefully mixing with a pipette. For phytase-subcloning, 0.15 pmol cleaved pET22b(+) and 0.2 pmol cleaved appA, 0.22 pmol phyY or 0.25 pmol phyX were combined. Resulting mixtures were incubated at room temperature for 5 minutes prior to transformation.

For hybridization during domain recombination experiments, a constant molar ratio of one to four (Fragments A1, B1 and C1; each 0.09 pmol and each -5.4 kB in size including the linearized pET22b(+)-vector to 12 fragments: A2-A5, B2-B5 & C2-C5; each 0.36 pmol and each 0.17 - 0.45 kB;) proved to be best to ensure efficient hybridization (Figure 5). The molar ratio of 0.09 pmol and 0.36 pmol also resulted in very good recombination and transformation results (see Results section). All 15 DNA fragments (five domains of each of the three phytase genes) were pooled on ice (Figure 5) and subsequently heated (70°C; 3 min; Eppendorf Gradient Cycler (Darmstadt, Germany)) to avoid preferential hybridization due to the order in pipetting. After incubation at room temperature (20°C; 5 min), 3 µl of the hybridization reaction were transformed without further purification or ligation steps into chemically competent *E*. *coli* BL21-Gold (DE3) cells (50 µl) following a standard protocol (Hanahan et al., 1991). Transformation mixtures were spread on agar plates with Luria Bertani (LB) medium supplemented with ampicillin (100 mg/ml) and incubated overnight (37°C).

### DNA-sequencing

Colonies carrying the pET22b(+)-vector with shuffled phytase domains were directly transferred into 96-well master plates, grown over night in LB medium (100 µl) with ampicillin (100 mg/ml) at 37°C, and stored (-80°C after addition of glycerol (100 µl; 50 vol%)). 96-well plates containing solid agar (GATC Biotech (Konstanz, Germany), were inoculated with clones harboring the pET22b(+)-vector with shuffled phytase chimeras and send to GATC Biotech (Konstanz, Germany) for sequencing (sequencing oligonucleotides: P33 and P34; Figure 7). Clone Manager 9 Professional Edition software (Scientific & Educational Software, Cary, NC, USA) was used for analyzing all sequencing data.

### Results

Three phytases, appA of Escherichia coli (A), phyY of Yersinia mollaretii (B) and phyX of Hafnia sp. LU11047 (C) were chosen to serve as model genes for development and validation the Domain-PLICing method. The number of crossover points was set to four within the targeted genes since most proof of concepts in gene shuffling methods have been performed with one to four crossover points. Domain-PLICing comprises four steps: 1) DNA-Fragment amplification by PCR using primers with complementary phosphorothioated nucleotides at the 5'-end, 2) Chemical cleavage of the PCR products in an iodine/ethanol solution for generating single-stranded overhangs (12 nt), 3) Random DNA-fragment assembly to full-length chimeric genes through hybridization at room temperature and 4) Transformation into competent host cells without further DNA purification or enzymatic DNA-ligation steps (Figure 5).

### Combinatorial DNA-fragment assembly by Domain-PLICing

All phytases were subcloned into the pET22b(+)-vector by PLICing as preparatory step for domain-shuffling experiments. The vector-backbone together with the N-terminal portion of the gene is regarded as domain A1, B1 and C1 for the three phytase genes (Figure 5). A sequence alignment of the three phytase proteins was used to select four crossover-sites for recombination. Only four conserved amino acids at potential crossover sites are necessary to design gene specific complementary oligonucleotides with twelve phosphorothioated nucleotides at the 5'-end (Figure 7). One additional crossover site near the STOP-codon was chosen to shuffle in total five protein domains of the three phytases (Figure 5). In a 19 cases, incorporation of silent mutations in the PCR primers was necessary to ensure complementary single-stranded 5'-overhangs for efficient hybridization (Figure 7). The 15 DNA-fragments for domain-shuffling were generated and purified in parallel as described in the Material and Methods section (Figure 5, Step 1). Domain-PLICing relies on the specific hybridization of single stranded 5'-ends of multiple DNA-fragments and a vector backbone as described for Phosphorothioate-based Ligase-Independent Gene Cloning (PLICing) which was developed as cloning technology for single (mutated) genes (Blanusa et al., 2010). For Domain-PLICing such overhangs were generated by chemical cleavage of the phosphorothioate bonds with iodine in an alkaline ethanol solution (Figure 5, Step 2). Those bonds were incorporated into the DNA-fragments beforehand during PCR-amplification of each DNA-fragment since phosphorothioated primers with 12 consecutive phosphorothioated nucleotides were employed (Figure 7). Key challenge for an oriented assembly of five fragments was a heat incubation protocol which enables oriented hybridization of all DNA-fragments to full plasmid size (Figure 5, Step 3). The heat incubation protocol ensures an efficient assembly of phytase chimeras consisting of five fragments and ten nicked DNA positions, even under transformation conditions. The latter could be achieved by a 70°C heat incubation (3 min; PCR-Cycler), cooling to 20°C (5 min; PCR- Cycler) and subsequent incubation on ice until transformation yielding 1100 clones (transformation efficiency: 0.51 x 10⁴ cfu/µg DNA, see Material and Methods).

### Sequencing of the chimeric phytase library

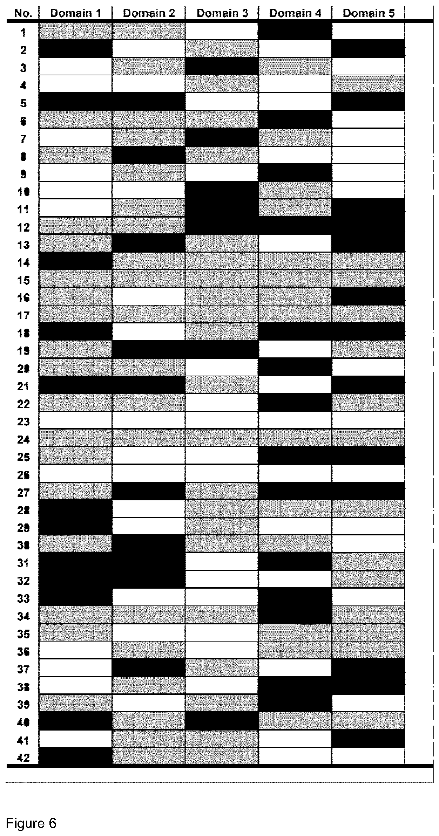

As a first control 25 clones were randomly picked and colony PCR performed. In all 25 cases a full length gene of correct size was obtained as PCR product (see Material and Methods). Encouraged by these results, 42 clones were randomly picked and both DNA strands were sequenced. Figure 6 shows the diverse pattern of chimeric phytase genes. All 42 genes contained the five expected domains in the correct order, proofing an efficient assembly through oriented hybridization. In none of the sequenced clones a domain was missing or occurring more than once. No preferential occurrence of any DNA-fragment of the three phytase genes could be detected during analyses of the domains 1, 2, 4 and 5 (domain 1: appA, 18; phyY, 12; phyX, 12 / domain 2: appA, 19; phyY, 13; phyX, 10 / domain 4: appA, 14; phyY, 14; phyX, 14 / domain 5: appA, 14; phyY, 15; phyX, 13). phyX-domain 3 from phytase A can be regarded as underrepresented (domain 3: appA, 21; phyY, 14; phyX, 7) in the chimeric library since phyX-domain 3 occurred only seven times instead of 14 times in an ideal case. From 42 sequenced clones, five were not recombined encoding the phytase gene appA (3x) and phyY (2x). The latter can be attributed likely to incomplete *Dpn*I digestion of the template DNA prior to chemical cleavage for overhang generation. In addition, two identical chimeric phytase genes occurred twice. Interestingly, no mutations and no frameshifts were observed around the five crossover sites, which would decrease the overall quality of the chimeric library. Within the chimeric genes transition mutations could be observed, which correspond to the error-rate of 2.2 x 10⁻⁵ of the employed *Taq* DNA polymerase (Fermentas, St. Leon-Rot, Germany).

## Claims

1. Nucleic acid joining method, comprising the steps of
a) providing a set of at least three nucleic acid fragments to be joined, wherein the nucleic acid fragments comprise pairwise compatible ends, and wherein at least two of the nucleic acid fragments are double stranded at least in the region of the respective compatible end, and wherein each counter-strand of the respective compatible end comprises nucleotides configured for non-enzymatic removal,
b) non-enzymatically removing of such nucleotides configured for non-enzymatic removal,
c) mixing of the nucleic acid fragments under hybridizing conditions,
wherein the nucleotides configured for non-enzymatic removal comprise a phosphorothioate backbone, and wherein the compatible ends have a length of 5 to 25 nucleotides, and wherein the number of nucleotides configured for non-enzymatic removal for each compatible end region is 5 to 25, and wherein at least one fragment comprises a double-stranded section after performing of step b).

2. Nucleic acid joining method according to claim 1, wherein in step c) at least two nucleic acid fragments are mixed in a molar ratio of 20:1 to 1:20.

3. Nucleic acid joining method according to claim 2, wherein step b) comprises exposing the fragments to iodine and ethanol.

4. Nucleic acid alteration method, comprising the steps of
a) providing a template nucleic acid, and providing primers for amplification of a template section of the template nucleic acid, wherein one or all primers have nucleotides configured for non-enzymatic removal,
b) amplification of the template section of the template nucleic acid to obtain a nucleic acid fragment,
c) repeating steps a) and b) to obtain at least two nucleic acid fragments,
d) performing a nucleic acid joining method according to any of the previous claims on the nucleic acid fragments of step c), and, where only two nucleic acid fragments are obtained in step c), introducing at least one further nucleic acid fragment,
wherein the nucleotides configured for non-enzymatic removal comprise a phosphorothioate backbone, and wherein the compatible ends have a length of 5 to 25 nucleotides, and wherein the number of nucleotides configured for non-enzymatic removal for each compatible end region is 5 to 25, and wherein at least one fragment comprises a double-stranded section after performing of a step b) as defined in claim 1.

5. Nucleic acid alteration method according to claim 4, wherein the product obtained by joining of the nucleic acid fragment differs from at least one of the templates and/or from a reference nucleic acid by one or more of
- a point mutation,
- the order and/or number and/or orientation of sequence sections,
- operative linkage to a transcription and/or translation influencing site,
- the order and/or number and/or length of transcribed and/or translated sequence sections.

6. Nucleic acid alteration method according to claim 5, wherein at least one first primer differs in at least one nucleotide from the corresponding section of the template nucleic acid.

7. Transformation method, comprising the steps of
a) performing a nucleic acid joining or alteration method according to any one of claims 1 to 6, and
b) transforming a target cell with the joined nucleic acid obtained in step a).

8. Transformation method according to claim 7, wherein the joined fragments form a cloning vector.

9. Use of at least three nucleic acid fragments as defined in any one of claims 1 to 6 for creating at least one difference relative to a template or reference nucleic acid, wherein difference is independently selected from:
- a point mutation,
- a difference in the order and/or number and/or orientation of sequence sections,
- a difference in operative linkage to a transcription and/or translation influencing site,
- a difference in the order and/or number and/or length of transcribed and/or translated sequence sections.

10. Kit for joining at least 3 nucleic acid fragments, comprising
- for each fragment, two amplification primers having nucleotides configured for non-enzymatic removal, and
- one or more non-enzymatic nucleotide removal agents,
wherein the nucleotides configured for non-enzymatic removal comprise a phosphorothioate backbone, wherein the nucleic acid fragments comprise pairwise compatible ends and wherein said compatible ends have a length of 5 to 25 nucleotides, and wherein the number of nucleotides configured for non-enzymatic removal for each compatible end region is 5 to 25, and wherein at least one fragment comprises a double-stranded section after performing of a step b) as defined in claim 1.

## Patentansprüche

1. Verfahren zur Verknüpfung von Nukleinsäuren, umfassend die Schritte
a) Bereitstellen eines Sets von mindestens drei zu verbindenden Nukleinsäurefragmenten, wobei die Nukleinsäurefragmente paarweise kompatible Enden umfassen, und wobei mindestens zwei der Nukleinsäurefragmente zumindest in der Region des jeweiligen kompatiblen Endes doppelsträngig sind, und wobei jeder Gegenstrang des jeweiligen kompatiblen Endes Nukleotide umfasst, die für enzymfreies Entfernen ausgestaltet sind,
b) enzymfreies Entfernen der Nukleotide, die für das enzymfreie Entfernen ausgestaltet sind,
c) Mischen der Nukleinsäurefragmente unter Hybridisierungsbedingungen,
wobei die Nukleotide, die für enzymfreies Entfernen ausgestaltet sind, ein Phosphorothioat-Rückgrat umfassen, und wobei die kompatiblen Enden eine Länge von 5 bis 25 Nukleotiden haben, und wobei die Anzahl an Nukleotiden, die für enzymfreies Entfernen ausgestaltet sind, für jede kompatible Endregion 5 bis 25 ist, und wobei mindestens ein Fragment nach Durchführung von Schritt b) einen doppelsträngigen Abschnitt umfasst.

2. Verfahren zur Verknüpfung von Nukleinsäuren nach Anspruch 1, wobei in Schritt c) mindestens zwei Nukleinsäurefragmente in einem molaren Verhältnis von 20:1 bis 1:20 gemischt werden.

3. Verfahren zur Verknüpfung von Nukleinsäuren nach Anspruch 2, wobei Schritt b) umfasst, dass die Fragmente Jod und Ethanol ausgesetzt werden.

4. Verfahren zum Verändern von Nukleinsäuren, umfassend die Schritte
a) Bereitstellen einer Vorlagenukleinsäure, und Bereitstellen von Primern zur Amplifikation eines Vorlageabschnittes der Vorlagenukleinsäure, wobei einer oder alle Primer Nukleotide, die für enzymfreies Entfernen ausgestaltet sind, aufweisen,
b) Amplifizieren des Vorlageabschnittes der Vorlagenukleinsäure, um ein Nukleinsäurefragment zu erhalten,
c) Wiederholen der Schritte a) und b), um mindestens 2 Nukleinsäurefragmente zu erhalten,
d) Durchführung eines Verfahrens zur Verknüpfung von Nukleinsäuren nach einem der vorangehenden Ansprüche an den Nukleinsäurefragmenten aus Schritt c), und, falls nur 2 Nukleinsäurefragmente in Schritt c) erhalten werden, Einbringen mindestens eines weiteren Nukleinsäurefragments,
wobei die Nukleotide, die für enzymfreies Entfernen ausgestaltet sind, ein Phosphorothioat-Rückgrat umfassen, und wobei die kompatiblen Enden eine Länge von 5 bis 25 Nukleotiden haben, und wobei die Anzahl an Nukleotiden, die für enzymfreies Entfernen ausgestaltet sind, für jede kompatible Endregion 5 bis 25 ist, und wobei mindestens ein Fragment nach Durchführung eines Schrittes b), wie in Anspruch 1 definiert, einen doppelsträngigen Abschnitt umfasst.

5. Verfahren zum Verändern von Nukleinsäuren nach Anspruch 4, wobei sich das Produkt, das durch das Verknüpfen des Nukleinsäurefragments erhalten wurde, von mindestens einer der Vorlagen und/oder von einer Referenznukleinsäure durch eine oder mehrere der Folgenden unterscheidet:
- eine Punktmutation,
- die Anordnung und/oder Anzahl und/oder Orientierung von Sequenzabschnitten,
- operative Verknüpfung mit einem die Transkription und/oder Translation beeinflussenden Bereich,
- die Anordnung und/oder Anzahl und/oder Länge an transkribierten und/oder translatierten Sequenzabschnitte.

6. Verfahren zum Verändern von Nukleinsäuren nach Anspruch 5, wobei sich mindestens ein erster Primer in mindestens einem Nukleotid von dem entsprechenden Abschnitt der Vorlagenukleinsäure unterscheidet.

7. Verfahren zur Transformation, umfassend die Schritte
a) Durchführen eines Verfahrens zur Verknüpfung oder zum Veränderung von Nukleinsäuren nach einem der vorangehenden Ansprüche 1 bis 6, und
b) Transformieren einer Zielzelle mit der verbundenen Nukleinsäure, die in Schritt a) erhalten wurde.

8. Verfahren zur Transformation nach Anspruch 7, wobei die verknüpften Fragmente einen Klonierungsvektor bilden.

9. Verwendung von mindestens 3 Nukleinsäurefragmenten, wie in einem der Ansprüche 1 bis 6 definiert, zum Herstellen von mindestens einem Unterschied in Relation zu einer Vorlage- oder Referenznukleinsäure, wobei der Unterschied unabhängig ausgewählt ist aus:
- einer Punktmutation,
- einem Unterschied in der Anordnung und/oder Anzahl und/oder Orientierung von Sequenzabschnitten,
- einem Unterschied in der operativen Verknüpfung zu einem die Transkription und/oder Translation beeinflussenden Bereich,
- einem Unterschied in der Anordnung und/oder Anzahl und/oder Länge an transkribierten und/oder translatierten Sequenzabschnitten.

10. Kit zur Verbindung von mindestens 3 Nukleinsäurefragmenten, umfassend
- für jedes Fragment zwei Amplifikationsprimer, die Nukleotide beinhalten, die für enzymfreies Entfernen ausgestaltet sind, und
- ein oder mehrere Substanzen zum enzymfreien Entfernen von Nukleotiden,
wobei die Nukleotide, die für enzymfreies Entfernen ausgestaltet sind, ein Phosphorothioat-Rückgrat umfassen, wobei die Nukleinsäurefragmente paarweise kompatible Enden umfassen und wobei besagte kompatiblen Enden eine Länge von 5 bis 25 Nukleotiden haben, und wobei die Anzahl an Nukleotiden, die für enzymfreies Entfernen ausgestaltet sind, für jede kompatible Endregion 5 bis 25 ist, und wobei mindestens ein Fragment nach Durchführung eines Schrittes b), wie in Anspruch 1 definiert, einen doppelsträngigen Abschnitt umfasst.

## Revendications

1. Procédé d'assemblage d'acides nucléiques, comprenant les étapes consistant à
a) fournir un ensemble d'au moins trois fragments d'acide nucléique à joindre, dans lequel les fragments d'acide nucléique comprennent des extrémités compatibles par paires, et dans lequel au moins deux des fragments d'acide nucléique sont à double brin au moins dans la région de l'extrémité compatible respective, et dans lequel chaque contre-brin de l'extrémité compatible respective comprend des nucléotides configurés pour une élimination non enzymatique,
b) éliminer de façon non enzymatique des nucléotides configurés pour l'élimination non enzymatique,
c) mélanger les fragments d'acide nucléique dans des conditions d'hybridation,
dans lequel les nucléotides configurés pour une élimination non enzymatique comprennent une épine dorsale phosphorothioate, et dans lequel les extrémités compatibles présentent une longueur de 5 à 25 nucléotides, et dans lequel le nombre de nucléotides configurés pour une élimination non enzymatique, pour chaque région d'extrémité compatible, est compris entre 5 et 25, et dans lequel au moins un fragment comprend une section à double brin après la mise en oeuvre de l'étape b).

2. Procédé d'assemblage d'acides nucléiques selon la revendication 1, dans lequel, à l'étape c), on mélange au moins deux fragments d'acide nucléique dans un rapport molaire compris entre 20 : 1 et 1 : 20.

3. Procédé d'assemblage d'acides nucléiques selon la revendication 2, dans lequel l'étape b) comprend l'exposition des fragments à l'iode et à l'éthanol.

4. Procédé d'altération d'acides nucléiques, comprenant les étapes consistant
a) à fournir un acide nucléique modèle et à fournir des amorces pour l'amplification d'une section modèle de l'acide nucléique modèle, dans lequel une ou l'ensemble des amorces ont des nucléotides configurés pour une élimination non enzymatique,
b) à amplifier la section modèle de l'acide nucléique modèle pour obtenir un fragment d'acide nucléique,
c) à répéter les étapes a) et b) pour obtenir au moins 2 fragments d'acide nucléique,
d) à mettre en oeuvre un procédé d'assemblage d'acides nucléiques selon l'une quelconque des revendications précédentes sur les fragments d'acide nucléique de l'étape c) et, si seulement 2 fragments d'acide nucléique sont obtenus à l'étape c), à introduire au moins un autre fragment d'acide nucléique,
dans lequel les nucléotides configurés pour une élimination non enzymatique comprennent une épine dorsale phosphorothioate, et dans lequel les extrémités compatibles présentent une longueur de 5 à 25 nucléotides, et dans lequel le nombre de nucléotides configurés pour une élimination non enzymatique, pour chaque région d'extrémité compatible, est compris entre 5 et 25, et dans lequel au moins un fragment comprend une section à double brin après la mise en oeuvre d'une étape b) telle que définie dans la revendication 1.

5. Procédé d'altération d'acides nucléiques selon la revendication 4, dans lequel le produit obtenu en joignant le fragment d'acide nucléique diffère d'au moins l'un des modèles et/ou d'un acide nucléique de référence par un(e) ou plusieurs de ceux qui suivent:
- une mutation ponctuelle,
- l'ordre et/ou le nombre et/ou l'orientation de sections de séquence,
- la liaison opérationnelle à un site influant sur la transcription et/ou la traduction,
- l'ordre et/ou le nombre et/ou la longueur de sections de séquence transcrites et/ou traduites.

6. Procédé d'altération d'acides nucléiques selon la revendication 5, dans lequel au moins une première amorce diffère dans au moins un nucléotide de la section correspondante de l'acide nucléique modèle.

7. Procédé de transformation comprenant les étapes consistant à
a) mettre en oeuvre un procédé d'assemblage ou d'altération d'acides nucléiques selon l'une quelconque des revendications 1 à 6, et à
b) transformer une cellule cible avec l'acide nucléique joint obtenu à l'étape a).

8. Procédé de transformation selon la revendication 7, dans lequel les fragments joints forment un vecteur de clonage.

9. Utilisation d'au moins 3 fragments d'acide nucléique, tels que définis dans l'une quelconque des revendications 1 à 6, pour créer au moins une différence par rapport à un acide nucléique modèle ou de référence, dans laquelle la différence est choisie indépendamment parmi:
- une mutation ponctuelle,
- une différence dans l'ordre et/ou le nombre et/ou l'orientation de sections de séquence,
- une différence dans la liaison opérationnelle à un site influant sur la transcription et/ou la traduction,
- une différence dans l'ordre et/ou le nombre et/ou la longueur de sections de séquence transcrites et/ou traduites.

10. Kit pour joindre au moins 3 fragments d'acide nucléique, comprenant,
- pour chaque fragment, deux amorces d'amplification ayant des nucléotides configurés pour une élimination non enzymatique et
- un ou plusieurs agents d'élimination non enzymatique de nucléotides,
dans lequel les nucléotides configurés pour une élimination non enzymatique comprennent une épine dorsale phosphorothioate, dans lequel les fragments nucléique comprennent des extrémités compatibles par paires et dans lequel lesdites extrémités compatibles présentent une longueur de 5 à 25 nucléotides, et dans lequel le nombre de nucléotides configurés pour une élimination non enzymatique, pour chaque région d'extrémité compatible, est compris entre 5 et 25, et dans lequel au moins un fragment comprend une section à double brin après la mise en oeuvre d'une étape b) telle que définie dans la revendication 1.
